# FASCICULE DE BREVET EUROPEEN

(11) **EP 0 759 934 B1**
(45) Date de publication et mention de la délivrance du brevet: **14.04.1999**
(21) Numéro de dépôt: 95920949.5
(22) Date de dépôt: 17.05.1995
(51) Int. Cl.: C07K 7/08, A61K 38/08, C12P 21/02

(54) **PEPTIDES ANTAGONISTES DE LA NEUROTENSINE**
PEPTIDISCHE NEUROTENSIN-ANTAGONISTEN
NEUROTENSIN ANTAGONIST PEPTIDES

(30) Priorité: 20.05.1994 FR 9406167
(43) Date de publication de la demande: 05.03.1997
(73) Titulaire: RHONE-POULENC RORER S.A., 92160 Antony (FR)
(72) Inventeur: CLERC, François-Frédéric, F-92330 Sceaux (FR); DUBROEUCQ, Marie-Christine, F-95880 Enghien-les-Bains (FR); HELYNCK, Gérard, F-94600 Choisy-le-Roi (FR); LEBOUL, Jean, F-91400 Gometz-la-Ville (FR); MARTIN, Jean-Paul, F-92700 Colombes (FR)
(74) Mandataire: Savina, Jacques
(86) Numéro de dépôt international: FR9500643
(87) Numéro de publication internationale: WO9532218

(56) Documents cités:
- US-A- 4 439 359
- TETRAHEDRON LETTERS, vol.31, no.45, 29 Octobre 1990, OXFORD GB pages 6581 - 6584 A G BROWN & P D EDWARDS 'Synthesis of analogues of the biphenomycin antibiotics'

## Description

La présente invention concerne des dérivés d'acides aminés de formule : 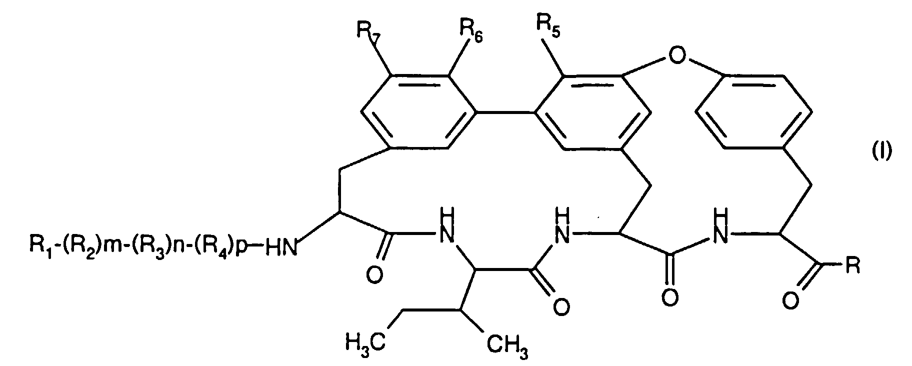 leur préparation, leurs sels et les médicaments les contenant.

Dans la formule (I),
R représente un radical hydroxy, alkyloxy, phénylalkyloxy ou -NH-CH₂-COOH,
R₁ représente un atome d'hydrogène, un radical adamantylacétyle, adamantylcarbonyle, norbornylacétyle, norbornylphénoxycarbonyle, benzoyle, nicotinoyle, 4-phénylbenzoyle, 4-tert-butylbenzoyle, 2-pyrrolidinecarbonyle ou un groupe protecteur d'une fonction amine,
R₂ représente un résidu Arg ou Lys,
R₃ représente un résidu Arg ou Lys,
R₄ représente un résidu Pro,
m, n et p, identiques ou différents représentent un nombre égal à 0 ou 1, étant entendu que lorsque R₁ est un atome d'hydrogène, la somme m+n+p est au moins égale à 1
R₅ et R₆ sont identiques et représentent un radical hydroxy ou méthoxy et
R₇ représente un atome d'hydrogène, de chlore, de brome ou d'iode ou un radical nitro.

La présente invention englobe également les équivalents des composés de formule (I) dans lesquels une ou plusieurs liaisons peptidiques (CO-NH) entre deux résidus d'acide aminé sont remplacées par des liaisons -CH₂-NH et/ou la liaison peptidique (CO-NH) entre les résidus d'acide aminé R₂ et R₃ est remplacée par une liaison CH=CH.

Dans les définitions qui précédent et celles qui suivent, les radicaux et portions alkyle et alkyloxy contiennent 1 à 4 atomes de carbone en chaîne droite ou ramifiée, Arg signifie arginine, Lys signifie lysine, Pro signifie proline, Fmoc signifie 9-fluorénylméthoxycarbonyle, Pmc signifie 2,2,5,7,8-pentaméthylchroman-6-sulfonyle, Boc signifie tert-butoxycarbonyle.

Dans la formule (I), chaque résidu d'acide aminé peut être dans la configuration L ou D.

Comme groupe protecteur de la fonction amine, on utilise, de préférence, les groupes 9-fluorénylméthoxycarbonyle, tertbutoxycarbonyle, acétyle, pivaloyle et benzyloxycarbonyle dont le phényle est éventuellement substitué par halogène, alkyle, alkyloxy ou nitro.

Les composés peptidiques de formule (I) pour lesquels la somme m+n+p est égale à au moins 1 peuvent être préparés par action d'un dérivé de formule : 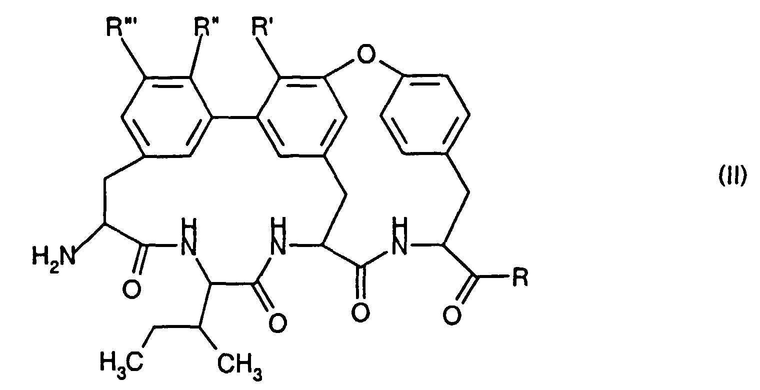 dans laquelle R a les mêmes significations que dans la formule (I), R' et R" sont identiques et représentent chacun un radical hydroxy ou méthoxy et R"' représente un atome d'hydrogène, de chlore, de brome ou d'iode ou un radical nitro sur un dérivé de formule :

R₁-(R₂)m-(R₃)n-(R₄)p-OH (III)

dans laquelle R₁ représente un radical adamantylacétyle, adamantylcarbonyle, norbornylacétyle, norbornylphénoxycarbonyle, benzoyle, nicotinoyle, 4-phénylbenzoyle, 4-tert-butylbenzoyle, 2-pyrrolidinecarbonyle ou un groupe protecteur d'une fonction amine, R₂, R₃, R₄, n, m et p ont les mêmes significations que dans la formule (I), la somme m+n+p étant égale à au moins 1, éventuellement suivie d'une déprotection de la fonction amine terminale pour obtenir les composés pour lesquels R₁ représente un atome d'hydrogène et/ou éventuellement lorsque R représente un radical alkyloxy ou phénylalkyloxy, d'une déprotection de la fonction carboxy pour obtenir les composés pour lesquels R représente un radical hydroxy.

Le couplage des dérivés de formules (II) et (III) s'effectue par toute méthode connue de l'homme du métier pour condenser un dérivé aminé et un peptide.

Il est particulièrement avantageux d'utiliser le dérivé de formule (III) sous forme activée. Comme forme activée, on peut citer le produit de réaction du dérivé de formule (III) avec la N-hydroxysuccinimide, le N-hydroxybenzotriazole ou l'hexafluorométhylphosphate de 2-[1H-benzotriazol-1-yl]-1,1,3,3-tétraméthyluronium qui peut être préparé au sein d'un solvant inerte tel qu'un amide comme le diméthylformamide, la N-méthylpyrrolidine-2-one, un solvant chloré tel que le chloroforme ou le chlorure de méthylène, un éther comme le tétrahydrofuranne ou un mélange de ces solvants, à une température comprise entre 15 et 60°C, en présence de tamis moléculaire 4Å ou d'un agent de condensation tel que le dicyclohexylcarbodiimide ou encore un ester de pentafluorophényle qui peut être préparé selon la méthode décrite par L. KISFALUDY et coll., Synthesis, 325-327 (1983).

La condensation du produit de formule (II) sur le produit activé de formule (III) s'effectue généralement dans les mêmes conditions de température et de milieu réactionnel que celles décrites précédemment pour la préparation de la forme activée du dérivé de formule (III) éventuellement en présence de pyridine ou d'une amine telle que la diisopropyléthylamine. Ces réactions peuvent être faites sans isoler le produit activé.

Il peut être avantageux d'effectuer cette réaction en phase solide, sur une résine. Les résines préférées sont les résines 4-hydroxyméthylphénoxyméthyl-copolystyrène 1% divinylbenzène (HMP, Applied Biosystems, WANG, J. Amer. Chem. Soc., 95, 1328, (1973)), chlorotritylchlorure-copolymère polystyrène-1% divinylbenzène (Novabiochem).

La déprotection éventuelle de la fonction amine terminale peut être effectuée selon les techniques habituelles de déprotection des amines telles que celles décrites par T.W. GREENE, Protective Groups in Organic Synthesis, John Wiley, New York. On peut par exemple opérer au sein d'un solvant inerte tel que le diméthylformamide ou un solvant chloré tel que le dichlorométhane, en présence de pipéridine, à une température voisine de 20-25°C.

La déprotection éventuelle de la fonction carboxy peut être effectuée par toute méthode connue de l'homme du métier permettant de passer d'une fonction ester carboxylique à une fonction carboxy. De préférence, on opère au sein d'un solvant inerte tel que le dioxanne, l'eau ou un mélange de ces solvants, en présence de lithine, à une température voisine de 0°C.

Le dérivé de formule (II) pour lequel R"' représentent un atome d'hydrogène, R, R' et R" représentent un radical hydroxy peut être préparé par fermentation d'actinomycètes A9738 (CBS 162.94) et isolement du produit.

La fermentation s'effectue selon les procédés classiques de fermentation. De préférence, on utilise comme milieu de culture un milieu comprenant de la peptone, un extrait de levure, un extrait de viande, du glucose, du carbonate de calcium, du chlorure de sodium et de l'agar. Cette fermentation est réalisée de préférence à une température comprise entre 25 et 30°C. Le moût est ensuite extrait et la phase aqueuse ultrafiltrée et chromatographiée plusieurs fois.

Les dérivés de formule (II) pour lesquels R représente un radical alkyloxy ou phénylalkyloxy peuvent être préparés par estérification du composé de formule (II) correspondant pour lequel R représente un radical hydroxy.

Cette estérification s'effectue, de préférence, par action d'un alcool R-OH pour lequel R représente un radical alkyle ou phénylalkyle soit en présence de chlorure de thionyle selon la méthode décrite par M.E. JUNG et coll., Tetrahedron Letters, 30 (32), 4211-4 (1989), soit en présence d'acide chlorhydrique gazeux ou d'acide sulfurique, à une température comprise entre 0 et 25°C, soit lorsque R représente un radical benzyloxy, en présence d'acide paratoluène sulfonique selon la méthode décrite par T.E. WALKER et coll., J. Org. Chem., 51, 1175-9 (1986). L'ester tert-butylique peut également être obtenu par action d'isobutène selon le procédé décrit dans le brevet US 3496219.

Les dérivés de formule (II) pour lesquels R représente un radical -NH-CH₂-COOH peuvent être préparés par action d'un dérivé de formule (II) correspondant pour lequel R représente un radical hydroxy sur la glycine.

Cette réaction s'effectue dans les conditions décrites précédemment pour la condensation des dérivés de formule (III) sur les dérivés de formule (II).

Les dérivés de formule (II) pour lesquels R' et R" représentent des radicaux méthoxy peuvent être préparés par action d'un composé de formule (II) correspondant pour lequel R' et R" représentent des radicaux hydroxy sur le triméthylsilyldiazométhane. Lorsque R représente un radical hydroxy non protégé, on obtient un dérivé de formule (II) pour lequel R, R' et R" représentent des radicaux méthoxy.

Cette réaction s'effectue au sein d'un alcool aliphatique inférieur (méthanol, éthanol par exemple), à une température voisine de 20°C.

Les dérivés de formule (II) pour lesquels R"' représente un radical nitro peuvent être préparés par nitration d'un dérivé de formule (II) correspondant pour lequel R"' représente un atome d'hydrogène.

Cette nitration s'effectue par toute méthode de nitration connue. De préférence, on fait réagir l'acide nitrique, au sein de l'acide acétique, à une température voisine de 20°C.

Les dérivés de formule (II) pour lesquels R"' représente un atome de chlore peuvent être préparés par chloration d'un dérivé de formule (II) correspondant pour lequel R"' représente un atome d'hydrogène.

Cette réaction s'effectue par toute méthode connue de chloration. De préférence, on fait réagir le 2,3,4,5,6,6-hexachloro-2,4-cyclohexadiène-1-one au sein d'un mélange diméthylformamide-tétrachlorure de carbone, à une température voisine de 20°C selon la méthode décrite par M. LEMAIRE et coll., Janssen Chimica Acta, 5(1), 3-8 (1987).

Les dérivés de formule (II) pour lesquels R"' représente un atome de brome peuvent être préparés par bromation d'un dérivé de formule (II) correspondant pour lequel R"' représente un atome d'hydrogène.

Cette réaction s'effectue par toute méthode connue de bromation. De préférence, on fait réagir le brome, en présence d'acétate de sodium, au sein de l'acide acétique, à une température voisine de 20°C.

Les dérivés de formule (II) pour lesquels R"' représente un atome d'iode peuvent être préparés par iodation d'un dérivé de formule (II) correspondant pour lequel R"' représente un atome d'hydrogène.

Cette réaction s'effectue par toute méthode connue de iodation. De préférence, on fait réagir un iodure de métal alcalin (iodure de sodium ou de potassium par exemple), en présence d'un réactif tel que le 1,2,4,6-tétrachloro-3α,6α-diphénylglycouril, au sein d'un alcool aliphatique inférieur tel que le méthanol, à une température voisine de 20°C.

Les dérivés de formule (III) sont commercialisés ou peuvent être préparés par application ou adaptation des méthodes décrites par S. DOULUT et coll., Peptide Research, 5 (1), 30-38 (1992; J. COUDER et coll., Int. J. Peptide Res., 41, 181-184 (1993); V.K. SKUKLA et coll., Can. J. Physiol. Pharmacol., 71, 211-216 (1993); M.H. MICHAEL et coll., J. Chem. Soc. Perkin I, 307-314 (1982); T.E. CHRISTOS et coll., Bioorganic Médicinal Chemistry Letters, 3 (6), 1035-1040 (1993), dans les brevets WO 93/00359 et WO 91/02750 et dans les exemples.

Les composés de formule (I) pour lesquels la somme m+n+p est au moins égale à 1, R₁ représente un atome d'hydrogène ou un groupe protecteur d'une fonction amine peuvent également être préparés par action d'un dérivé de formule (II) correspondant sur successivement un dérivé (R₄)p-OH et/ou (R₃)n-OH et/ou (R₂)m-OH dans lesquels R₄, R₃ et R₂ ont les mêmes significations que dans la formule (I) dont les restes amino sont de préférence protégés suivie éventuellement d'une déprotection.

Cette réaction s'effectue dans les conditions décrites dans la littérature pour la chimie des peptides telles que celles décrites précédemment pour le couplage des dérivés de formule (II) et de formule (III).

Les composés de formule (I) pour lesquels la somme m+n+p est égale à 0, R₁ représente un groupe protecteur de la fonction amine peuvent être préparés par toute méthode connue de l'homme de l'art de protection d'une fonction amine ne modifiant pas le reste de la molécule. En particulier lorsque R₁ représente un radical 9-fluorénylméthoxycarbonyle on fait réagir un dérivé de formule (II) sur le carbonate de 9-fluorénylméthyle et de N-succinyle.

Cette réaction s'effectue généralement au sein d'un solvant inerte tel que le dioxanne, en présence d'une solution aqueuse de carbonate de sodium, à une température voisine de 20°C.

Lorsque R₁ représente un radical tertbutoxycarbonyle, acétyle, pivaloyle et benzyloxycarbonyle dont le phényle est éventuellement substitué par halogène, alkyle, alkyloxy ou nitro on fait réagir un dérivé de formule (II) sur un dérivé R₁-Cl dans lequel R₁ représente un radical tertbutoxycarbonyle, acétyle, pivaloyle ou benzyloxycarbonyle dont le phényle est éventuellement substitué par halogène, alkyle, alkyloxy ou nitro.

Cette réaction s'effectue de préférence au sein d'un solvant inerte tel que le diméthylformamide, en présence d'une base telle qu'une trialkylamine (diisopropyléthylamine par exemple), à une température voisine de 20°C.

Les composés de formule (I) pour lesquels la somme m+n+p est égale à zéro et R₁ représente un radical adamantylacétyle, adamantylcarbonyle, norbornylacétyle, norbornylphénoxycarbonyle, benzoyle, nicotinoyle, 4-phénylbenzoyle, 4-tert-butylbenzoyle, 2-pyrrolidinecarbonyle peuvent également être préparés par action d'un dérivé de formule (II) pour lequel R₁ représente un atome d'hydrogène sur un chlorure R₁-Cl dans lequel R₁ représente un radical adamantylacétyle, adamantylcarbonyle, norbornylacétyle, norbornylphénoxycarbonyle, benzoyle, nicotinoyle, 4-phénylbenzoyle, 4-tert-butylbenzoyle, 2-pyrrolidinecarbonyle.

Cette réaction s'effectue de préférence au sein d'un solvant inerte tel que le diméthylformamide, en présence d'une base telle qu'une trialkylamine (diisopropyléthylamine par exemple), à une température voisine de 20°C.

Les composés de formule (I) peuvent être purifiés par les méthodes connues habituelles, par exemple par chromatographie ou extraction.

Il est entendu pour l'homme du métier que, pour la mise en oeuvre des procédés décrits précédemment, il peut être nécessaire d'introduire des groupes protecteurs des fonctions amino et carboxy afin d'éviter des réactions secondaires puis de déprotéger. De tels groupes protecteurs sont décrits par T.W. GREENE, Protective Groups in Organic Synthesis, John Wiley, New York et dans les exemples.

Les composés de formule (I) pour lesquels R représente un radical hydroxy peuvent être éventuellement transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'un acide organique sur un composé de formule (I), dans un solvant. Le sel formé est séparé par les méthodes habituelles.

Les composés de formule (I) pour lesquels R₁ représente un atome d'hydrogène peuvent être éventuellement transformés en sels d'addition avec un acide minéral ou organique par action d'un tel acide au sein d'un solvant organique tel qu'un alcool, une cétone, un éther ou un solvant chloré.

Ces sels font également partie de l'invention.

Comme exemples de sels pharmaceutiquement acceptables, peuvent être cités les sels d'addition avec les acides minéraux ou organiques (tels que acétate, trifluoroacétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, pipéridine, benzylamine, N-benzyl-α-phénéthylamine, choline, arginine, leucine, lysine, N-méthyl glucamine).

Les composés de formule (I) présentent des propriétés pharmacologiques intéressantes. Ces composés sont des antagonistes de la neurotensine et sont donc utiles pour traiter ou prévenir des désordres associés à la neurotensine.

C'est ainsi que ces composés peuvent être utilisés pour le traitement ou la prévention des psychoses, des troubles anxieux, de la dépression, des troubles cognitifs, de la neurodégénération, des attaques de panique, de la maladie de Parkinson, de la maladie d'Alzheimer, de la schizophrénie, de l'autisme, de la diskynésie tardive, du syndrome du colon irritable, de la pancréatite aiguë, des ulcères, des désordres de la motilité intestinale, de certaines tumeurs sensibles à la neurotensine, dans le sevrage aux traitements chroniques et abus d'alcool ou de médicaments, des phénomènes allergiques et inflammatoires, des troubles cardiovasculaires et respiratoires dans lesquels la neurotensine est impliquée et de l'asthme.

L'affinité des composés de formule (I) vis-à-vis de la neurotensine a été mesurée par leur capacité à déplacer la liaison de la neurotensine tritiée de ses récepteurs présents dans une préparation membranaire brute de cortex cérébral de cobaye. Le test est basé sur celui décrit par M. GOEDERT et coll., Brain Research, 304, 71-81 (1984) : des cobayes mâles Dunkin-Hartley (200-300 g) sont sacrifiés par décapitation et le cerveau rapidement prélevé. Toutes les étapes suivantes sont réalisées à 4°C. Le cortex cérébral est disséqué, pesé et homogénéisé dans 5 ml de tampon Tris-HCl (50 mM, pH 7,4) par gramme de tissu avec un polytron (force 6 pendant 15 secondes). L'homogénéisat est centrifugé à 48000 g pendant 15 minutes et le culot obtenu lavé deux fois dans le même tampon. Le culot final est homogénéisé dans 3 ml de tampon par gramme de tissu initial et gardé sous forme d'aliquots (environ 20 mg de protéines par ml) à -80°C jusqu'à utilisation. Le contenu protéique est mesuré selon la méthode de BRADFORD, Anal. Biochem., 72, 248-254 (1976). Le test de liaison est réalisé dans du tampon Tris-HCl 50mM pH 7,4 contenant 0,4% d'albumine de sérum bovin et 0,1 mM de bacitracine en présence de 0,15 mg de protéines par ml et 0,8nM de [³H]neurotensine pendant 15 minutes à 25°C. La liaison non spécifique est déterminée en présence de 1µM de neurotensine 1-13. La réaction est arrêtée par filtration et la radioactivité retenue sur le filtre mesurée par scintillométrie. Les produits sont étudiés en gamme de concentrations afin de déterminer la concentration inhibitrice de 50% de la liaison spécifique. Les composés de formule (I) présentent dans ce test une CI₅₀ inférieure à 15 µM.

Les composés de formule (I) présentent une faible toxicité compatible avec leur utilisation comme principe actif de médicaments.

Les exemples suivants illustrent l'invention.

Pour les spectres de R.M.N., la nomenclature utilisée est celle de la Protein Data Bank, à l'exception des radicaux méthyle qui sont appelés M. Les résidus aminoacides des résidus macrocycliques des composés de formule (I) et (II) sont appelés X-TYR1 (amino-acide N terminal); X-ILE2; X-TYR3 et X-TYR4 (amino-acide C terminal). Les résidus aminoacides de la chaîne sont appelés R suivi du nom de l'aminoacide et de sa position dans la chaîne.

Un échantillon de la souche actinomycètes A9738 a été déposé et enregistré auprès du Centraalbureau voor Schimmel culturen (CBS) à Baarn (Pays Bas) dans les conditions du traité de Budapest, le 22 mars 1994 sous le numéro CBS 162.94.

### EXEMPLE 1

On solubilise 61 mg du dérivé de formule (II) pour lequel R"' représente un atome d'hydrogène, R, R' et R" représentent des radicaux hydroxy, 64 mg de Fmoc-Arg-Pro-OH et 13 mg de N-hydroxybenzotriazole dans 1 ml de diméthylformamide contenant du tamis moléculaire 4 Å. Après 15 heures, on ajoute 10 µl de pyridine et 21 mg de dicyclohexylcarbodiimide et on laisse réagir 72 heures à 40°C. On ajoute ensuite 15 ml d'eau et le précipité ainsi formé est collecté sur verre fritté n° 4. Le solide est délité par 10 ml de méthanol puis filtré. Le filtrat est étendu à 15 ml puis additionné d'eau (85 ml). La solution est soumise à une chromatographie liquide haute performance avec une colonne de silice greffée octadécyle (250x10 mm) au débit de 3,5 ml/minute. L'élution est effectuée à l'aide d'un gradient linéaire d'eau 0,07% d'acide trifluoroacétique à acétonitrile-eau (70/30 en volumes) 0,07% acide trifluoroacétique. Des fractions de 1,7 ml sont collectées. Celles contenant le produit recherché sont rassemblées et évaporées sous pression réduite. On isole ainsi 0,6 mg du composé de formule (I) pour lequel R₁-(R₂)ₘ-(R₃)ₙ-(R₄)ₚ représente Fmoc-Arg-Pro-, R, R₅ et R₆ représentent des radicaux hydroxy et R₇ représente un atome d'hydrogène sous forme d'une poudre beige [Spectre de masse sur perkin Elmer API III en ionspray : M/z 1092 MH⁺ 1114 MNa⁺; Spectre de R.M.N. (400MHz, DMSO, δ en ppm) : R-FMOC1: HA1, HA2 4,22; HB 4,30; aromatiques 7,70; 7,31; 7,40; 7,89; R-ARG2 : NH 7,90; HA 4,20; HB1, HB2 1,45, 1,22; HG1, HG2 1,45; HD1, HD2 2,97; guanidine large; R-PRO3 - HA 4,42, HB1, HB2 1,83, 1,88; HG1, HG2 1,88, 1,88; HD1, HD2 3,60, 3,40; X-TYR1 : NH 6,98; HA 4,69; HB1, HB2 2,92; HD1 6,90; HD2 6,70; HE1 6,68; X-ILE2 : NH 8,27; HA 4,05; HB 1,60; MG1 0,82; HG21, HG22 1,15, 1,55; MD 0,83; X-TYR3 : NH 8,65; HA 3,58; HB1, HB2 2,88, 2,40; HD1 6,57; HD2 5,47; X-TYR4 : NH 4,27; HA 4,18, HB1, HB2 2,48, 2,52; HD1 7,16; HD2 7,31; HE1 7,51; HE2 6,78].

Le peptide Fmoc-Arg-Pro-OH peut être synthétisé en phase solide, en utilisant une stratégie de synthèse Fmoc sur un appareil Applied Biosystems 431A en utilisant les cycles "standard Fmoc" fournis par le constructeur avec la N-méthylpyrrolidine-2-one comme solvant. La déprotection des fonctions alpha-amine est réalisée par une solution de pipéridine à 20% dans la N-méthylpyrrolidine-2-one pendant 20 minutes à chaque étape de synthèse. Le peptide est synthétisé sur 0,25 mmol de résine de Wang (Wang, J. Amer. Chem. Soc., 95, 1328 (1973)) 4-hydroxyméthylphénoxyméthyl-copolystyrène 1% divinylbenzène (HMP, Applied Biosystems). On forme l'anhydride symétrique de la Fmoc-Pro-OH (1mmol) par réaction pendant 20 minutes avec 0,5 mmol de dicyclohexylcarbodiimide dans 1,3 ml de N-méthylpyrrolidine-2-one et 1,8 ml de dichlorométhane. Après 13 minutes de réaction, 0,36 ml de diméthylaminopyridine à 0,1 M dans le diméthylformamide sont ajoutés. Après élimination par filtration de la dicyclohexylurée formée, l'anhydride symétrique est mis en réaction pendant 30 minutes avec la résine. La fonction amine de la proline est déprotégée par réaction pendant 20 minutes avec une solution à 20% de pipéridine dans la N-méthylpyrrolidine-2-one. L'ester N-hydroxybenzotriazolyle de la Fmoc-Arg(Pmc)-OH est formé par réaction de 1 mmol de Fmoc-Arg(Pmc)-OH dans 4,1 ml de N-méthylpyrrolidine-2-one en présence de 1 mmol de N-hydroxybenzotriazole et 1 mmol de dicyclohexylcarbodiimide pendant 20 minutes. Après élimination de la dicyclohexylurée formée, l'ester est mis en réaction pendant 30 minutes avec la résine. On obtient ainsi une résine sur laquelle est greffé le groupe Fmoc-Arg(Pmc)-Pro. Le peptide est clivé de la résine par traitement pendant 1 heure et 30 minutes dans 10 ml d'acide trifluoracétique, 0,75 g de phénol, 0,25 ml d'éthanedithiol, 0,5 ml de thioanisole et 0,5 ml d'eau pour 100 mg de peptidyl-résine. Après élimination de la résine par filtration, la phase liquide est concentrée à l'évaporateur rotatif pendant 30 minutes sous pression réduite (4 kPa). Le peptide est ensuite précipité par addition d'un mélange éther tert-butylméthylique-éther de pétrole (4/1 en volumes) et récupéré par centrifugation. Le peptide est repris par un volume minimal d'acide trifluoroacétique, précipité par addition d'un mélange d'éther tert-butylméthylique et d'éther de pétrole (4/1 en volumes) et récupéré par centrifugation, cette opération est répétée deux fois. Le peptide est ensuite lavé par 30 ml d'un mélange d'éther tert-butylméthylique et d'éther de pétrole (4/1 en volumes), récupéré par centrifugation et séché sous vide (4 kPa). Le peptide est utilisé tel quel pour les étapes suivantes de la synthèse.

Le composé de formule (II) pour lequel R"' représente un atome d'hydrogène, R, R' et R" représentent des radicaux hydroxy peut être obtenu de la manière suivante : dans un erlenmeyer de 2 litres, un milieu de 250 ml contenant 5g/l de peptone, 5 g/l d'extrait de levure, 15 g/l de glucose, 5 g/l d'extrait de viande, 3 g/l de carbonate de calcium, 5 g/l de chlorure de sodium et 1 g/l d'agar est ensemencé par une gélose inclinée d'actinomycètes A9738 (CBS 162.94) sous forme de suspension de spores. Après agitation à 140 t/mn dans un agitateur thermostaté à 28°C pendant 72 heures, la totalité de la culture est transférée stérilement dans un fermenteur primaire rempli de 60 litres du même milieu. Le fermenteur est agité à 300 t/mn, aéré à 5 m³/h et thermostaté à 28°C pendant 44 heures. La totalité de la culture est alors transférée stérilement dans un fermenteur de production avec 450 litres de milieu stérilisé 40 minutes à 122°C (distillers 5 g/l, haricots 40 g/l, glucose 5 g/l, huile de soja 10 g/l, chlorure de sodium 5 g/l et chlorure de cobalt 20 mg/l) puis maintenue à une température de 28°C pendant 94 heures, sous agitation à 250 t/mn et aéré à raison de 15 m³/h. Le moût (487 litres, pH 7,8) est ensuite agité avec 5% de clarcel commercialisé par CECA puis filtré pour séparer le mycélium du filtrat. Le filtrat (300 litres) est extrait par 2 fois 100 litres d'acétate d'éthyle et la phase aqueuse (250 litres) est ultrafiltrée par une membrane d'ultrafiltration dont le seuil de coupure est de 20 kD juqu'à obtenir un volume de rétentat de 40 litres. L'ultrafiltrat (210 litres) est percolé sur une colonne en acier inoxydable (20x60 cm) contenant 30 litres de résine Duolite S861 commercialisé par Rhom et Haas avec un débit de 30 l/h. La colonne est rincée avec 80 litres d'eau puis la chromatographie est effectuée par un gradient par palier méthanol-eau avec un débit de 30 l/h et des fractions de 10 litres (50/50 en volumes pour les fractions 1 et 2, 60/40 en volumes pour les fractions 3 à 8, 80/20 en volumes pour les fractions 9 et 10 et 100% de méthanol pour les fractions 11 et 12). Les fractions 3 à 6 sont rassemblées, le méthanol est éliminé sous pression réduite (3,4 kPa) puis la phase aqueuse est lyophilisée. On obtient ainsi 12,4 g d'une poudre jaune. Ce lyophilisat est délité par 600 ml de méthanol refroidi à 4°C, l'insoluble est écarté et la solution est concentrée sous pression réduite (3,4 KPa) pour donner 4 g d'extrait sec. Cet extrait est solubilisé par 110 ml d'une solution méthanol-eau (1/10 en volumes) et chromatographié sur une colonne en acier inoxydable (5x60 cm) remplie de silice greffée C18 Matrex (20 µ, 60Å) commercialisée par Amicon à l'aide d'un système GILSON. Les fractions de 50 ml sont collectées avec un collecteur de fractions Pharmacia et le débit est de 50 ml/minute. Le profil de gradient utilisé est le suivant : en 30 minutes, gradient linéaire de méthanol-eau 10/90 en volumes à 40/60 en volumes, palier de 30 minutes à 40/60 en volumes puis gradient linéaire de 40/60 en volumes à 100% de méthanol en 40 minutes. Les fractions 21 à 29 sont rassemblées et concentrées sous pression réduite (3,4 kPa) à un volume de 10 ml. Cette solution est déposée au sommet d'une colonne en verre (5x30cm) remplie de polyamide SC6 Macherey Nagel. L'élution s'effectue à l'eau. 50 fractions de volumes variables (20 à 70 ml) sont collectées. Après avoir écarté les fractions 1 à 12 (environ 1100 ml), les fractions 13 à 25 (600 ml) sont rassemblées et déposées sur une colonne en verre de 5 cm de diamètre et contenant 250 ml de DEAE Biogel A (Bio-Rad). L'élution s'effectue à l'eau. Les premiers 900 ml d'effluent et d'éluat sont écartés puis le produit recherché est élué à la suite avec 2 litres d'eau. Ces 2 litres sont percolés sur un réservoir Analytichem international de 75 ml (2,5 cm de diamètre interne) contenant 40 ml de silice greffée C18 Bondesil 40 µ commercialisée par Analytichem International. L'effluent est écarté et le produit est élué au méthanol. La solution méthanolique est concentrée sous pression réduite pour donner 60 mg de produit de formule (II) pour lequel R"' représente un atome d'hydrogène, R, R' et R" représentent des radicaux hydroxy pur sous forme d'une poudre légérement jaune [spectre IR : bandes caractéristiques (cm⁻¹) à 3395 (OH phénol), 3300 (NH), 2970 (CH3), 2935 (CH2), 2875 (CH3, CH2), 3125 à 2125 (OH acide), 1670 (C=O acide et amide), 1585 à 1500 (noyaux benzèniques), 1235 (CO éther aromatique et phénol), 865 et 835 (CH aromatiques; Spectre de masse (Finnegan TSQ46 en D/IC avec comme gaz réactant l'ammoniac) M/z=634 MNH₄⁺, 617 MH⁺; Spectre R.M.N. (400 MHz, DMSO d6, δ en ppm) : X-TYR1 : NH2 large; HA 4,14; HB1, HB2 3,05, 3,20; HD1 7,10; HD2 6,80; HE1 6,81; X-ILE2 : NH 8,38; HA 4,20; HB 1,58; MG1 0,88; HG21, HG22 1,20, 1,51; MD 0,84; X-TYR3 : NH 8,48; HA 3,63; HB1, HB2 2,42, 2,88; HD1 6,60; HD2 5,59; X-TYR4 : NH 4,22; HA 4,26; HB1, HB2 3,02, 3,47; HD1 7,24, HD2 7,26, HE1 7,46; HE2 6,78].

### EXEMPLE 2

Une solution de 60 mg de Fmoc-Arg-Arg-Pro-OH, 16 mg de N-hydroxysuccinimide et 20 mg de dicyclohexylcarbodiimide dans 1,5 ml de diméthylformamide est chauffée pendant 15 heures à 50°C. On ajoute ensuite 50 mg du dérivé de formule (II) pour lequel R"' représente un atome d'hydrogène, R, R' et R" représentent des radicaux hydroxy et une goutte de pyridine. et laisse réagir 72 heures à une température de 50°C. Le mélange réactionnel est déposé en tête d'une colonne de silice (2,6x6cm) équilibrée dans du dichlorométhane. Après un lavage au dichlorométhane (45 ml) la colonne est percolée avec un mélange acétate d'éthyle-acide acétique-eau (60/12/10 en volumes).

Des fractions de 4,5 ml sont collectées. Les fractions contenant le produit recherché sont rassemblées et évaporées à sec sous pression réduite (3,4 kPa) pour donner 10 mg d'une poudre beige. Cette poudre est soumise à une chromatographie liquide haute performance avec une colonne de silice greffée octadécyle (250x10 mm) au débit de 3,5 ml/minute. L'élution est effectuée à l'aide d'un gradient linéaire d'eau 0,07% d'acide trifluoroacétique à acétonitrile-eau (70/30 en volumes) 0,07% d'acide trifluoroacétique. Des fractions de 1,7 ml sont collectées. Celles contenant le produit recherché sont rassemblées et évaporées sous pression réduite pour donner 5 mg du composé de formule (I) pour lequel R₁-(R₂)ₘ-(R₃)ₙ-(R₄)ₚ représente Fmoc-Arg-Arg-Pro-, R, R₅ et R₆ représentent des radicaux hydroxy et R₇ représente un atome d'hydrogène sous forme d'une poudre légèrement beige [Spectre de masse sur Perkin Elmer API III en ionspray : M/z : 1248 MH⁺, 624,8 (M+2H)⁺⁺; Spectre de R.M.N. (400MHz, DMSO, δ en ppm : R-Fmoc1 : HA1, HA2 4,28, 4,32; HB 4,32; aromatiques 7,68; 7,32; 7,42; 7,88; R-ARG2 : NH 7,30; HA 4,08; HB1, HB2 1,65, 1,50; HG1, HG2 1,50; HD1, HD2 3,15; guanidine 7,35, 6,90; R-ARG3 : NH 7,98; HA 4,48; HB1, HB2 1,50; HG1, HG2 1,50; HD1, HD2 3,10; guanidine 7,22, 6,90; R-PRO4 : HA 4,38; HB1, HB2 1,85, 1,95; HG1, HG2 1,85, 1,95; HD1, HD2 3,45, 3,61; X-TYR1 : NH 6,90; HA 4,92; HB1, HB2 2,98; HD1 6,90; HD2 6,90; HE1 6,80; X-ILE2 : NH 8,12; HA 4,13; HB 1,63; MG1 0,85; HG21, HG22 1,22, 1,51; MD 0,83; X-TYR3 : NH 8,48; HA 3,62; HB1, HB2 2,65, 2,70; HD1 6,58; HD2 5,52; X-TYR4 - NH 4,22; HA 4,32; HB1, HB2 3,20, 3,35; HD1 7,28; HD2 7,30; HE1 7,51; HE2 6,78].

La synthèse du peptide Fmoc-Arg-Arg-Pro-OH est conduite comme décrit pour le peptide Fmoc-Arg-Pro-OH dans l'exemple 1. Une résine sur laquelle on a greffé au préalable un groupe Fmoc-Arg(Pmc)-Pro- comme décrit à l'exemple 1 est déprotégée de son groupe protecteur Fmoc par de la pipéridine à 20% dans la N-méthylpyrrolidine-2-one pendant seulement 3 minutes pour limiter la formation d'un dérivé dicétopipérazinique correspondant. La résine est lavée extensivement par de la N-méthylpyrrolidine-2-one. Parallèlement l'ester N-hydroxybenzotriazolyle de la Fmoc-Arg(Pmc)-OH est formé par réaction de 1 mmol de Fmoc-Arg(Pmc)-OH dans 2,1 ml de N-méthylpyrrolidine-2-one, 1 ml de N-hydroxybenzotriazole 1M dans la N-méthylpyrrolidine-2-one et 1 ml de dicyclohexylcarbodiimide 1M dans la N-méthylpyrrolidine-2-one pendant 20 minutes. Après élimination de la dicyclohexylurée formée, cet ester est mis en réaction pendant 30 minutes avec la résine greffée. On obtient ainsi une résine sur laquelle est greffé le groupe Fmoc-Arg(Pmc)-Arg(Pmc)-Pro-. Le peptide Fmoc-Arg-Arg-Pro-OH est clivé de la résine et purifié de la même manière que décrit dans l'exemple 1 pour Fmoc-Arg-Pro-OH.

### EXEMPLE 3

On dissout 7 mg du produit de l'exemple 2 dans 180 µl de diméthylformamide et on ajoute 20 µl de pipéridine. La solution se trouble rapidement et un précipité apparaît. Le précipité est collecté par centrifugation et lavé par deux fois 200 µl d'acétonitrile. On obtient ainsi 1 mg du composé de formule (I) pour lequel R₁-(R₂)ₘ-(R₃)ₙ-(R₄)ₚ représente H-Arg-Arg-Pro-, R, R₅ et R₆ représentent des radicaux hydroxy et R₇ représente un atome d'hydrogène sous forme de sel de pipéridine [Spectre de masse sur Perkin Elmer API III en ionspray : M/z 1026 MH⁺].

### EXEMPLE 4

A une solution de 62 mg du composé de formule (II) pour lequel R"' représente un atome d'hydrogène, R, R' et R" représentent des radicaux hydroxy et de 52,5 µl de diisopropyléthylamine dans 3ml de diméthylformamide, on ajoute 50,3 mg de N-Fmoc-L-prolinate de pentafluorophényle et agite 18 heures à une température voisine de 25°C. On dilue alors avec 25 ml d'acétate d'éthyle et 25 ml d'eau, puis acidifie avec une solution aqueuse d'acide chlorhydrique 1N. Après décantation, la phase organique est lavée par 3 fois 25 ml d'eau et séchée sur sulfate de magnésium. Après filtration, lavage et évaporation du filtrat (2 kPa), on obtient 80 mg de solide. Ce solide est purifié par chromatographie liquide haute performance sur une colonne LiChroprep diol (MERCK) de 2,5 cm de diamètre et 31 cm de longueur, en éluant à un débit de 10 ml par minute, d'abord avec 200 ml du mélange dichlorométhane-éthanol (95/5 en volumes), puis 200 ml du mélange dichlorométhane-éthanol (93/7 en volumes). Les fractions contenant le composé attendu sont réunies et évaporées à sec (2 kPa), pour donner 25 mg du composé de formule (I), pour lequel R₁-(R₂)ₘ-(R₃)ₙ-(R₄)ₚ représente Fmoc-Pro-, R, R₅ et R₆ représentent des radicaux hydroxy et R₇ représente un atome d'hydrogène sous forme d'un solide blanc [Spectre de masse (Autospec VG FISONS en LSIMS) : M/z = 936 (M + H)⁺, M/z = 714 (M - Fmoc + 2H)⁺; Spectre infrarouge (KBr): 3390, 2965, 2930, 2875, 3100 à 2250, 1680, 1585, 1450, 1505, 835, 760, 740 cm⁻¹ ; Spectre de RMN (600 MHz; DMSO; TEMP = 350K, δ en ppm : R-Fmoc1 : HA1, HA2 4,20, 4,32; HB 4,32; aromatiques 7,83; 7,28; 7,48; 7,88; R-PRO2 : HA 4,22; HB1, HB2 1,74, 2,08; HG1, HG2 1,78, 1,95; HD1, HD2 3,28, 3,35; X-TYR1 : NH 7,62; HA 4,71; HB1, HB2 2,91; 3,06; HD1 6,89; HD2 6,84; HE1 6,68; X-ILE2 - NH 7,95; HA 4,15; HB 1,62; MG1 0,82; HG21, HG22 1,12, 1,47; MD 0,79; X-TYR3 : NH 8,24; HA 3,62; HB1, HB2 2,46; 2,70; HD1 6,62; HD2 5,52; X-TYR4: NH 4,22; HA 4,26; HB1, HB2 3,10, 3,38; HD1 7,18; HD2 7,25; HE1 7,45; HE2 6,78].

### EXEMPLE 5

9 mg du composé de l'exemple 4 sont dissous dans 0,5 ml du mélange dichlorométhane-pipéridine (90/10 en volumes). Après 96 heures à une température voisine de 25°C, les solvants sont évaporés sous pression réduite (2 kPa). Le résidu est repris dans 0,5 ml d'eau, puis lavé par deux fois 1 ml d'éther diéthylique. La phase aqueuse est amenée à un pH voisin de 4 avec de l'acide acétique. La suspension obtenue est alors chromatographiée sur une colonne préparative de chromatographie liquide haute performance de silice greffée octadécyle Bio-Rad 100 Å de 25 cm de longueur et de 1 cm de diamètre avec un débit de 6 ml par minute. L'élution est effectuée à l'aide d'un gradient linéaire eau-acide trifluoroacétique (100/0,07 en volumes) à eau-acétonitrile-acide trifluoroacétique (63/37/0,07 en volumes), en 33 minutes. Des fractions de 3 ml sont recueillies. Les fractions contenant le produit recherché sont jointes, congelées à -80°C et lyophilisées (1 Pa) pour donner 0,6 mg du composé de formule (I) pour lequel R₁-(R₂)ₘ-(R₃)ₙ-(R₄)ₚ représente H-Pro-, R, R₅ et R₆ représentent des radicaux hydroxy et R₇ représente un atome d'hydrogène sous forme d'un solide blanc [Spectre de masse (Finnegan TSQ46 en désorpsion par ionisation chimique avec comme gaz réactant l'ammoniac): M/z = 714 (MH⁺); Spectre RMN (600 MHz; DMSO; TEMP =303 K; δ en ppm) : R-PRO1 : HA 4,10; HB1, HB2 1,77, 2,12; HG1, HG2 1,73; HD1, HD2 3,20; X-TYR1 : NH 8,13; HA 4,78; HB1, HB2 2,94; 3,03; HD1 6,78; HD2 6,78; HE1 6,71; X-ILE2 : NH 8,31; HA 4,10; HB 1,62; MG1 0,82; HG21, HG22 1,12; 1,47; MD 0,79; X-TYR3 : NH 8,66; HA 3,59; HB1, HB2 2,42; 2,69; HD1 6,58; HD2 5,49; X-TYR4 : NH 4,14; HA 4,31; HB1, HB2 3,10; 3,38; HD1 7,18; HD2 7,32; HE1 7,52; HE2 6,80].

### EXEMPLE 6

20 mg du composé de formule (II) pour lequel R représente un radical méthoxy, R"' représente un atome d'hydrogène, R' et R" représentent des radicaux hydroxy sont ajoutés à un mélange de 23,2 mg de trifluoroacétate de (Boc)₂Lys-Ψ [CH₂NH]-Lys(Boc)-Pro-OH, 11,4 mg d'hexafluorométhylphosphate de 2-[1H-benzotriazol-1-yl]-1,1,3,3-tétraméthyluronium et 16 µl de diisopropyléthylamine dans 3 ml de diméthylformamide, à une température voisine de 20°C. Le mélange est agité pendant 6 heures et 30 minutes à cette même température. Les solvants sont évaporés sous pression réduite (15 Pa). Le résidu est repris dans 5 ml de dichloroéthane et lavé par deux fois 1 ml d'eau, deux fois 1 ml d'une solution 0,1 M de monophosphate de sodium, deux fois 1 ml d'une solution 0,25 M d'hydrogénocarbonate de sodium et deux fois 1 ml d'eau. La phase organique est concentrée sous pression réduite (5,3 kPa). Le résidu est repris dans 2 ml d'acide trifluoroacétique aqueux (95/5 en volumes) à une température voisine de 20°C pendant 1 heure et 30 minutes. Après concentration à un volume d'environ 0,2 ml (5,3 kPa), le produit est précipité par addition de méthyl-tert-butyléther et collecté par centrifugation. On obtient ainsi 2,2 mg du composé de formule (I) pour lequel R₁-(R₂)ₘ-(R₃)ₙ-(R₄)ₚ représente H-Lys-Ψ[CH₂NH]-Lys-Pro-, R représente un radical méthoxy, R₅ et R₆ représentent des radicaux hydroxy et R₇ représente un atome d'hydrogène de couleur jaune clair [Spectre de masse sur Sciex API m en ionspray: M/z = 970 (MH⁺)].

Le composé de formule (II) pour lequel R représente un radical méthoxy, R"' représente un atome d'hydrogène, R' et R" représentent des radicaux hydroxy peut être obtenu de la manière suivante : on dissout 200 mg du composé de formule (II) pour lequel R R', R" représentent des radicaux hydroxy et R"' représente un atome d'hydrogène dans 20 ml de méthanol et ajoute 0,8 ml d'acide sulfurique concentré. La solution est agitée 18 heures à une température voisine de 20°C. On ajoute alors 10 g de Duolite S 861 (Rhom et Haas), dilue avec 200 ml d'eau et agite 30 minutes à une température voisine de 20°C. On filtre et lave la résine sur verre fritté avec 60 ml d'eau. On extrait ensuite le composé par élution avec 100 ml de méthanol. Après évaporation sous pression réduite (3,4 kPa), le résidu est purifié par chromatographie sur 170 ml de résine carboxyméthyl-Triacryl (SEPRACOR), en éluant avec de l'eau, et en recueillant des fractions de 10 ml. Les fractions contenant le composé recherché sont jointes et évaporées à sec pour donner 40 mg du composé de formule (II) pour lequel R représente un radical méthoxy, R"' représente un atome d'hydrogène, R' et R" représentent des radicaux hydroxy sous forme d'une poudre légèrement jaune [Spectre de masse sur Sciex API III en ionspray: M/z = 631 (MH⁺); Spectre infra-rouge (FMIR, dans le méthanol): 3390, 3275, 2960, 2875, 1740, 1660, 1585+ 1500, 1510, 1215, 865+ 835 cm⁻¹; Spectre RMN (400 MHz; DMSO; TEMP = 303 K; δ en ppm) : X-TYR1 : NH2 7,92; HA 4,78; HB1, HB2 3,01; 3,17; HD1 7,09; HD2 6,74; HE1 6,82; X-ILE2 : NH 8,63; HA 4,19; HB 1,62; MG1 0,82; HG21, HG22 1,18, 1,48; MD 0,79; X-TYR 3 : NH 8,73; HA 3,58; HB1, HB2 2,42, 2,62; HD1 6,51; HD2 5,50; X-TYR 4 : NH 4,22; HA 4,45; HB1, HB2 3,21; 3,39; HD1 7,12, HD2 7,36; HE1 7,58; HE2 6,82; OCH₃ 3,81].

Le composé (Boc)₂Lys-Ψ[CH₂NH]-Lys(Boc)-Pro-OH peut être préparé selon la méthode de S. DOULUT et coll., Peptide Research, 5 (1), 30-38 (1992), en faisant réagir le (Boc)₂Lysinal sur H-Lys(Boc)-Pro-OBg puis en saponifiant l'ester terminal (OBg ester = N-benzhydrylglycolamide ester: Tetrahedron, 44, 5101-5108 (1988)).

### EXEMPLE 7

37 mg du composé décrit à l'exemple 6 sont dissous dans 0,6 ml de dioxanne, 0,3 ml d'eau et 0,3 ml de solution de lithine 1M, à une température voisine de 0°C. Après 70 minutes d'agitation à une température voisine de 0°C, on dilue avec 10 ml d'acide acétique aqueux (10/90 en volumes) et on injecte le brut sur une colonne préparative de chromatographie liquide haute performance de silice greffée octadécyle Bio-Rad RSL 100 Å de 30 cm de longueur et de 1 cm de diamètre. L'élution est effectuée à l'aide d'un gradient linéaire d'eau-acide trifluoroacétique (100/0,07 en volumes) à eau-acétonitrile-acide trifluoroacétique (65/35/0,07 en volumes), en 30 minutes. Les fractions contenant le produit recherché sont jointes, congelées à -80°C et lyophilisées (1 Pa) pour donner 4,4 mg du composé de formule (I) pour lequel R₁-(R₂)ₘ-(R₃)ₙ-(R₄)ₚ représente H-Lys-Ψ[CH₂NH]-Lys-Pro-, R, R₅ et R₆ représentent des radicaux hydroxy et R₇ représente un atome d'hydrogène sous forme de poudre blanche [Spectre de masse sur Sciex API III en ionspray: M/z = 956 (MH⁺)].

### EXEMPLE 8

La synthèse en phase solide est effectuée au moyen du matériel de la société Shandon (groupe Life Science International) à l'exception du rotateur pour tubes à hémolyse. La résine est confinée dans des seringues d'extraction en phase solide de 3 ml en polyéthylène haute densité (PE-HD) munies de filtres en téflon. Ces seringues sont montées sur une vanne deux voies en téflon et fermées par un bouchon à ailettes à usage unique en PE-HD. L'agitation des seringues est réalisée sur un rotateur pour tubes à hémolyse. Les opérations de lavage et de filtration sont conduites sur une station de travail d'extraction en phase solide (enceinte à vide munie d'embouts luer, Shandon).

La synthèse est réalisée sur 50 µmol de résine en chimie Fmoc. Les couplages des acides aminés sont réalisés en traitant pendant 1 heure la résine par 250 µmol de l'acide aminé convenablement protégé en présence de 250 µmol de 2-(1H-benzotriazole-1-yl)-1,1,3,3-tétraméthyluronium hexafluorophosphate, 250 µmol de N-hydroxybenzotriazole et 750 µmol de diisopropyléthylamine dans 1,2 ml d'un mélange N-méthylpyrrolidine-2-one-diméthylformamide (1/1 en volumes). La déprotection du groupement Fmoc est réalisée par 3 traitements successifs de la résine pendant 1 minute, 1 minute et 20 minutes respectivement, avec 2 ml d'une solution de de pipéridine à 20% en volumes dans la N-méthylpyrrolidine-2-one.

On soumet successivement 50 µmol de résine Fmoc-Gly-[résine de Wang] (Wang et coll., J. Amer. Chem. Soc., 95, 1328, (1973)) aux traitements suivants :
- Déprotection du groupement Fmoc,
- Lavage par 5 fois 2 ml de N-méthylpyrrolidine-2-one,
- Couplage de 2 équivalents du composé de formule (II) pour lequel R, R' et R" représentent chacun un radical hydroxy et R"' représente un atome d'hydrogène dont l'amino est protégé par Fmoc,
- Lavage par 5 fois 2 ml de N-méthylpyrrolidine-2-one,
- Déprotection du groupement Fmoc,
- Lavage par 5 fois 2 ml de N-méthylpyrrolidine-2-one,
- Couplage de 5 équivalents de Fmoc-proline,
- Lavage par 5 fois 2 ml de N-méthylpyrrolidine-2-one,
- Déprotection du groupement Fmoc,
- Lavage par 5 fois 2 ml de N-méthylpyrrolidine-2-one,
- Couplage de 5 équivalents de Fmoc-arginine(Pmc),
- Lavage par 5 fois 2 ml de N-méthylpyrrolidine-2-one,
- Déprotection du groupement Fmoc,
- Lavage par 5 fois 2 ml de N-méthylpyrrolidine-2-one,
- Couplage de 5 équivalents de Fmoc-arginine(Pmc),
- Lavage par 5 fois 2 ml de N-méthylpyrrolidine-2-one,
le peptide est ensuite clivé par réaction dans 10 ml d'un mélange acide trifluoroacétique-phénol-éthanedithiol-thioanisole-eau (40/3/1/2/2 en volumes) pendant 90 minutes. La résine est éliminée par filtration. Le filtrat est concentré sous pression réduite au moyen d'un évaporateur rotatif équipé d'une pompe à membrane et d'un piège à carboglace pendant 1 heure, la température du bain étant maintenue à 45°C. Le volume final du concentrat est d'environ 1 ml. Le produit est précipité par addition de 15 ml de méthyl-tert-butyléther et recueilli par centrifugation. Le culot est solubilisé dans 1 ml d'acide trifluoroacétique, précipité par addition de 15 ml de méthyl-tert-butyléther puis lavé par 15 ml d'un mélange méthyl-tert-butyléther-éther de pétrole (2/1 en volumes) en présence de 0,2 ml d'acide trifluoroacétique. Le produit est séché sous pression réduite (3,5 kPa), puis purifié par chromatographie liquide à haute performance sur une colonne C₁₈ 100 Å (250 x 10 mm, Bio-Rad), en éluant avec un gradient de 20 à 40% d'acétonitrile contenant 0,07% en volumes d'acide trifluoroacétique dans l'eau contenant 0,07% en volumes d'acide trifluoroacétique à un débit de 6 ml/minute, en 30 minutes, puis lyophilisé. On obtient ainsi 3,4 mg de ditrifluoroacétate du composé de formule (I) pour lequel R représente un radical -NH-CH₂-COOH, R₁-(R₂)ₘ-(R₃)ₙ-(R₄)ₚ représente Fmoc-Arg-Arg-Pro-, R₅ et R₆ représentent des radicaux hydroxy et R₇ représente un atome d'hydrogène sous la forme d'une poudre blanche [Spectre de masse sur Sciex API III en ESMS (Electrospray Mass Spectrometry) : M/z = 1306 (MH)⁺, M/z = 653 (M+2H)²⁺].

### EXEMPLE 9

On traite 180 mg (170 µmol) de Boc-Arg(benzyloxycarbonyle)₂-Arg(benzyloxycarbonyle)₂-Pro-OH par 31 mg (150 µmol) de dicyclohexylcarbodiimide et 23 mg (170 µmol) de N-hydroxybenzotriazole dans 3 ml de diméthylformamide pendant 4 heures à une température voisine de 20°C. La dicyclohexylurée formée est ensuite filtrée sur un filtre Millex HV 0,45 µm (Millipore) et le filtrat est ajouté à une solution contenant 100 mg (162 µmol) du composé de formule (II) pour lequel R"' représente un atome d'hydrogène, R, R' et R" représentent des radicaux hydroxy et 0,025 ml (143 µmol) de diisopropyléthylamine dans 2,5 ml de diméthylformamide. La réaction est conduite pendant 24 heures à une température voisine de 20°C. Le solvant est éliminé grâce à un évaporateur centrifuge Speed Vac (Savant) équipé d'une pompe à palettes pendant 18 heures à une température voisine de 20°C. Le résidu est solubilisé dans 20 ml de dichlorométhane et la solution extraite successivement par 2 fois 4 ml d'eau distillée, 2 fois 4 ml d'une solution de dihydrogénophosphate de sodium 0,1 M dans l'eau distillée et 2 fois 4 ml d'eau distillée. On obtient ainsi le composé de formule (I) pour lequel R₁-(R₂)ₘ-(R₃)ₙ-(R₄)ₚ représente Boc-Arg(benzyloxycarbonyle)₂-Arg(benzyloxycarbonyle)₂-Pro-, R, R₅ et R₆ représentent des radicaux hydroxy et R₇ représente un atome d'hydrogène en solution dans le dichlorométhane. Cette solution est divisée en trois parts égales qui sont séchées. Une de ces parties est solubilisée dans 2 ml d'un mélange dichlorométhane-méthanol (1/1 en volumes). A cette solution on ajoute 100 mg de palladium sur charbon à 10% et 100 mg de formiate d'ammonium. La réaction est conduite pendant 100 minutes avec agitation périodique au vortex. Le milieu réactionnel est ensuite filtré sur un filtre Millex HV 0,45 µm (Millipore) et le filtre lavé par 5 ml de méthanol. Le solvant est évaporé sous pression réduite (2,6 kPa, 45°C, 30 minutes). Le produit est enfin purifié par chromatographie liquide à haute performance sur une colonne C₁₈ 100 Å (250x10 mm, Bio-Rad), en éluant avec un gradient de 15 à 40% d'acétonitrile contenant 0,07% d'acide trifluoroacétique (en volumes) dans l'eau contenant 0,07% d'acide trifluoroacétique (en volumes) à un débit de 6 ml/minute en 30 minutes, puis lyophilisé. On obtient ainsi 15,2 mg du composé de formule (I) pour lequel R₁-(R₂)ₘ-(R₃)ₙ-(R₄)ₚ représente Boc-Arg-Arg-Pro-, R, R₅ et R₆ représentent des radicaux hydroxy et R₇ représente un atome d'hydrogène sous la forme d'une poudre blanche [Spectre de masse sur Sciex API III en ESMS (Electrospray Mass Spectrometry) : M/z = 1126 (MH)⁺, M/z = 563 (M+2H)²⁺].

Le peptide Boc-Arg(benzyloxycarbonyle)₂-Arg(benzyloxycarbonyle)₂-Pro-OH peut être préparé de la manière suivante : le composé Boc-Arg(benzyloxycarbonyle)₂-OH est mis à réagir avec le prolinate de méthyle en présence de 2-(1H-benzotriazole-1-yl)-1,1,3,3-tétraméthyluronium hexafluorophosphate dans l'acétonitrile ou le diméthylformamide en utilisant la diisopropyléthylamine comme base pour obtenir un pH supérieur à 8, le groupement Boc est ensuite clivé par l'acide trifluoroacétique à 40% dans le dichlorométhane. Le composé Boc-Arg(benzyloxycarbonyle)₂-OH est mis à réagir avec ce produit, en présence de 2-(1H-benzotriazole-1-yl)-1,1,3,3-tétraméthyluronium hexafluorophosphate dans l'acétonitrile ou le diméthylformamide en utilisant la diisopropyléthylamine comme base pour obtenir un pH supérieur à 8. L'ester obtenu peut ensuite être saponifié par la lithine, à une température voisine de + 4°C.

### EXEMPLE 10

Une des 3 parties du composé de formule (I) pour lequel R₁-(R₂)ₘ-(R₃)ₙ-(R₄)ₚ représente Boc-Arg(benzyloxycarbonyle)₂-Arg(benzyloxycarbonyle)₂-Pro-, R, R₅ et R₆ représentent des radicaux hydroxy et R₇ représente un atome d'hydrogène obtenue à l'exemple 9 est traitée pendant 90 minutes par 5 ml d'un mélange acide trifluoroacétique-eau distillée (95/5 en volumes). Le milieu est ensuite amené à sec à l'évaporateur rotatif (2,6 kPa; 45°C; 45 minutes) et 15 ml d'eau distillée sont ensuite ajoutés au milieu. La suspension obtenue est congelée à -80°C puis lyophilisée (96 heures). Le produit est solubilisé dans 1 ml de diméthylformamide contenant 0,0625 ml de diisopropyléthylamine (0,36 mmol). On ajoute ensuite 50 µl (50 µmol) d'une solution de chlorure d'acide 1-adamantylacétique (obtenue par réaction de 38,8 mg (200 µmol) d'acide 1-adamantylacétique avec 17,2 µl (200 µmol) de chlorure d'oxalyle dans 200 µl de dichlorométhane en présence d'une goutte de diméthylformamide, pendant 15 minutes à une température voisine de 20°C) à la solution de peptide et la réaction est conduite à une température voisine de 20°C, pendant 1 heure. Le solvant est ensuite éliminé grâce à un évaporateur centrifuge Speed Vac (Savant) équipé d'une pompe à palettes pendant 18 heures à une température voisine de 20°C. Le résidu est solubilisé dans 2 ml d'un mélange dichlorométhane-méthanol (1/1 en volumes) puis additionné de 100 mg de palladium sur charbon à 10% et 100 mg de formiate d'ammonium. La réaction est conduite pendant 150 minutes avec agitation périodique au vortex. Le milieu réactionnel est filtré sur un filtre Millex HV 0,45 µm (Millipore) et le filtre lavé par 5 ml de méthanol. Le solvant est ensuite évaporé sous pression réduite (2,6 kPa, 45°C, 30 minutes). Le produit est enfin purifié par chromatographie liquide à haute performance sur une colonne C₁₈ 100 Å (250 x 10 mm, Bio-Rad), en éluant avec un gradient de 15 à 40% d'acétonitrile contenant 0,07% d'acide trifluoroacétique (en volumes) dans l'eau contenant 0,07% d'acide trifluoroacétique (en volumes), à un débit de 6 ml/minute en 30 minutes, puis lyophilisé. On obtient ainsi 2,9 mg de ditrifluoroacétate du composé de formule (I) pour lequel R₁-(R₂)ₘ-(R₃)ₙ-(R₄)ₚ représente 1-adamantylacétyl-Arg-Arg-Pro-, R, R₅ et R₆ représentent des radicaux hydroxy et R₇ représente un atome d'hydrogène, sous la forme d'une poudre blanche [Spectre de masse, sur Sciex API III en ESMS (Electrospray Mass Spectrometry) : M/z = 601,6 (M+2H)²⁺].

### EXEMPLE 11

Une des 3 parties du composé de formule (I) pour lequel pour lequel R₁-(R₂)ₘ-(R₃)ₙ-(R₄)ₚ représente Boc-Arg(benzyloxycarbonyle)₂-Arg(benzyloxycarbo nyle)₂-Pro-, R, R₅ et R₆ représentent des radicaux hydroxy et R₇ représente un atome d'hydrogène obtenue à l'exemple 9 est traitée pendant 90 minutes par 5 ml d'un mélange acide trifluoroacétique-eau distillée (95/5 en volumes). Le millieu est ensuite amené à sec à l'évaporateur rotatif (2,6 kPa, 45°C, 45 minutes). On ajoute 15 ml d'eau distillée au résidu et la suspension est congelée à -80°C puis lyophilisée (96 heures). Le produit obtenu est solubilisé dans 1 ml de diméthylformamide contenant 62,5 µl de diisopropyléthylamine (0,36 mmol). On ajoute ensuite 9,9 mg (50 µmol) de chlorure d'acide 1-adamantylcarboxylique à la solution de peptide et la réaction est conduite à une température voisine de 20°C pendant 1 heure. Le solvant est ensuite éliminé grâce à un évaporateur centrifuge Speed Vac (Savant) équipé d'une pompe à palettes pendant 18 heures à une température voisine de 20°C. Le résidu est solubilisé dans 2 ml d'un mélange dichlorométhane-méthanol (1/1 en volumes) puis additionné de 100 mg de palladium sur charbon à 10% et 100 mg de formiate d'ammonium. La réaction est conduite pendant 150 minutes avec agitation périodique au vortex. Le milieu réactionnel est ensuite filtré sur un filtre Millex HV 0,45 µm (Millipore) et le filtre lavé par 5 ml de méthanol. Le solvant est évaporé sous pression réduite. Le produit est enfin purifié par chromatographie liquide à haute performance sur une colonne C₁₈ 100 Å (250 x 10 mm, Bio-Rad) en éluant avec un gradient de 18 à 43% d'acétonitrile contenant 0,07% d'acide trifluoroacétique (en volumes) dans l'eau contenant 0,07% d'acide trifluoroacétique (en volumes), à un débit de 6 ml/minute en 30 minutes puis lyophilisé. On obtient ainsi 3,1 mg de ditrifluoroacétate du composé de formule (I) pour lequel R₁-(R₂)ₘ-(R₃)ₙ-(R₄)ₚ représente 1-adamantylcarbonyl-Arg-Arg-Pro-, R, ₅ et R6 représentent des radicaux hydroxy et R₇ représente un atome d'hydrogène sous la forme d'une poudre blanche [Spectre de masse, sur Sciex API m en ESMS (Electrospray Mass Spectrometry) : M/z = 594,5 (M+2H)²⁺].

### EXEMPLE 12

On traite 100 mg (85 µmol) de Fmoc-Arg(Pmc)-Arg(Pmc)-Pro-OH par 17,4 mg de dicyclohexylcarbodiimide (85 µmol) et 12,2 mg de N-hydroxybenzotriazole (90 µmol) dans 1,5 ml de diméthylformamide pendant 4 heures à une température voisine de 20°C. La dicyclohexylurée formée est ensuite filtrée sur un filtre Millex HV 0,45 µm (Millipore) et le filtre est lavé par 0,5 ml de diméthylformamide. On ajoute ensuite 0,45 ml du filtrat obtenu (correspondant à 19 µmol de Fmoc-Arg(Pmc)-Arg(Pmc)-Pro-OH) à 0,25 ml d'une solution contenant 16,7 mg (22,5 µmol) du composé de formule (II) pour lequel R, R₅ et R₆ représentent des radicaux hydroxy et R₇ représente un atome d'iode et 4 µl (23 µmol) de diisopropyléthylamine dans le diméthylformamide. La réaction est conduite pendant 3 heures à une température voisine de 20°C. Le solvant est éliminé grâce à un évaporateur centrifuge Speed Vac (Savant) équipé d'une pompe à palettes pendant 18 heures à une température voisine de 20°C. Le résidu est ensuite repris par 5 ml d'un mélange acide trifluoroacétique-phénol-éthanedithiol-thioanisole-eau (40/3/1/2/2 en volumes) et laissé à réagir pendant 90 minutes, à une température voisine de 20°C, sous agitation. Le mélange est ensuite concentré sur un évaporateur centrifuge RC10-10 (Jouan) équipé d'une pompe à palettes pendant 45 minutes (température de la chambre d'évaporation 50°C, piégeage des vapeurs à -90°C). Le concentrat obtenu (environ 1 ml) est additionné de 15 ml d'un mélange éther tert-butylméthylique-éther de pétrole (1/1 en volumes) pour précipiter le peptide. Le précipité est collecté par centrifugation et solubilisé par 1 ml d'acide trifluoroacétique. L'opération de précipitation est répétée 1 fois. Le peptide est séché sous pression réduite (3,5 kPa). Le produit est enfin purifié par chromatographie liquide à haute performance (CLHP) sur une colonne C₁₈ 100 Å (250 x 10 mm, Bio-Rad), en éluant avec un gradient de 22 à 47% d'acétonitrile contenant 0,07% d'acide trifluoroacétique (en volumes) dans l'eau contenant 0,07% d'acide trifluoroacétique (en volumes) à un débit de 6 ml/minute en 30 minutes puis lyophilisé. On obtient ainsi 10 mg de ditrifluoroacétate du composé de formule (I) pour lequel R₁-(R₂)ₘ-(R₃)ₙ-(R₄)ₚ représente Fmoc-Arg-Arg-Pro-, R, R₅ et R₆ représentent des radicaux hydroxy et R₇ représente un atome d'iode sous la forme d'une poudre blanche [Spectre de masse, sur Sciex API III en ESMS (Electrospray Mass Spectrometry) : M/z = 1374, (M+H)⁺, M/z = 687 (M+2H)²⁺].

Le composé de formule (II) pour lequel R, R' et R" représentent des radicaux hydroxy et R"' représente un atome d'iode peut être obtenu de la manière suivante : 740 mg du composé de formule (II) pour lequel R, R' et R" représentent des radicaux hydroxy et R"' représente un atome d'hydrogène sont dissous dans 60 ml de méthanol et additionnés de 180 mg d'iodure de sodium, puis de 133 mg de 1,2,4,6-tétrachloro-3α,6α-diphényl glycouril. La solution est agitée 45 minutes à une température voisine de 20°C. Les solvants sont alors évaporés sous pression réduite (2 kPa) et le brut réactionnel est fixé sur 10 g de gel de silice. On dépose ce mélange sur une colonne de 4 cm de diamètre contenant 150 g de gel de silice et élue avec le mélange acétate d'éthyle-acide acétique-eau (103/12/10 en volumes), en recueillant des fractions de 100 ml. Les fractions comprises entre 900 et 2400 ml sont jointes et concentrées à sec pour donner 734 mg d'un solide de couleur ocre. Ce solide est repris dans 20 ml d'eau et 100 ml d'acétate d'éthyle, tiédi à une température voisine de 40°C et refroidi à une température voisine de 20°C avant d'être filtré, lavé avec 3 ml d'eau, puis avec trois fois 20 ml d'acétate d'éthyle. On le sèche sous pression réduite (30 Pa), à 40°C, pour obtenir 387,7 mg du composé de formule (II), pour lequel R, R' et R" représentent des radicaux hydroxy et R"' représente un atome d'iode sous forme d'un solide de couleur beige fondant à une température supérieure à 260°C [Spectre de masse sur Autospec VG FISONS, en LIMS (Liquid Secondary Ion Mass Spectroscopy), matrice de glycérol-thioglycérol: M/z = 743 (MH)⁺;S PECTRE RMN (600 MHZ, DMSO, TEMP 303 K; δ en ppm) : X-TYR1 : HA 3,98; HB1,HB2 3,16, 2,80; HD1,HD2 7,34, 6,67; X-ILE2 : NH 8,51; HA 4,18; HB 1,62; MG1 0,84; HG21,HG22 1,49, 1,16; MD 0,82; X-TYR3 : NH 8,61; HA 3,64; HB1,HB2 2,66, 2,40; HD1,HD2 6,52, 5,29; X-TYR4 : NH 4,19; HA 4,27; HB1,HB2 3,39, 3,02; HD1,HD2 7,40, 7,22; HE1,HE2 7,10, 6,70].

Le composé Fmoc-Arg(Pmc)-Arg(Pmc)-Pro-OH peut être préparé de la manière suivante : on obtient une résine Fmoc-Pro-chlorotrityl en traitant 0,3 mmol de résine chlorotritylchlorure-copolymère polystyrène 1% divinylbenzène (Novabiochem) par 1 mmol de Fmoc-proline dans 3,5 ml d'un mélange dichlorométhane-diisopropyléthylamine (6/1 en volumes) pendant 30 minutes à une température voisine de 20°C. On ajoute ensuite 2 ml de méthanol et on laisse la réaction se poursuivre pendant 30 minutes à une température voisine de 20°C. La résine est ensuite lavée successivement par 3 fois 5 ml de méthanol et 3 fois 5 ml de dichlorométhane et séchée.

Le peptide Fmoc-Arg(Pmc)-Arg(Pmc)-Pro-OH est assemblé sur un appareil Applied Biosystems 431A en utilisant les cycles "standard Fmoc", fournis par le constructeur, en utilisant la N-méthylpyrrolidine-2-one comme solvant. La déprotection des fonctions alpha-amines est réalisée par de la pipéridine à 20% dans la N-méthylpyrrolidine-2-one pendant 20 minutes à chaque étape de la synthèse. La résine Fmoc-Pro-chlorotrityl est déposée dans le réacteur de l'appareil. Après déprotection de la fonction alpha-amine de la proline, l'ester N-hydroxybenzotriazolyle de la Fmoc-Arg(Pmc) est formé par réaction de 1 mmol de Fmoc-Arg(Pmc)-OH dans 2,1 ml de N-méthylpyrrolidine-2-one, 1 ml de N-hydroxybenzotriazole 1M dans la N-méthylpyrrolidine-2-one et 1 ml de dicyclohexylcarbodiimide 1M dans la N-méthylpyrrolidine-2-one pendant 20 minutes. Après élimination de la dicyclohexylurée formée, l'ester est mis en réaction pendant 30 minutes avec la résine. La fonction alpha-amino de l'arginine est ensuite déprotégée et l'ester N-hydroxybenzotriazolyle de la Fmoc-Arg(Pmc)-OH est couplé à la résine comme décrit précédemment. Le peptide Fmoc-Arg(Pmc)-Arg(Pmc)-Pro-OH est détaché de la résine en traitant celle-ci pendant 15 minutes par 10 ml d'un mélange acide acétique-trifluoroéthanol-dichlorométhane (1/2/7 en volumes) à une température voisine de 20°C. La résine est ensuite lavée successivement par 10 ml de dichlorométhane et 10 ml d'acétonitrile. Les phases organiques sont jointes et les solvants sont éliminés à l'évaporateur rotatif (2,6 kPA, 1 heure, 45°C). Le résidu est repris par 1 ml d'acétonitrile et additionné de 30 ml d'eau distillée. La suspension obtenue est congelée à -80°C et lyophilisée. On obtient ainsi 102 mg de Fmoc-Arg(Pmc)-Arg(Pmc)-Pro-OH

### EXEMPLE 13

3,06 g du composé de formule (II) pour lequel R, R' et R" représentent des radicaux hydroxy et R"' représente un atome d'hydrogène sont dissous dans 80 ml d'une solution aqueuse de carbonate de sodium à 5%, auxquels on ajoute 1,8 g de carbonate de 9-fluorénylméthyle et de N-succinyle puis 40 ml de dioxanne. La réaction se poursuit pendant 5 heures à une température voisine de 20°C. Le mélange réactionnel est extrait par 2 fois 100 ml d'acétate d'éthyle; les phases organiques sont éliminées puis la phase aqueuse est acidifiée à pH entre 1 et 2 par une solution d'acide chlorhydrique concentré. On extrait par 3 fois 100 ml d'acétate d'éthyle et les phases organiques sont combinées, séchées par du sulfate de sodium, filtrées sur gel de silice puis évaporées sous pression réduite (2 kPa). Le précipité est délité par 300 ml d'un mélange méthyltert-butyléther-chlorure de méthylène (1/1 en volumes) puis séché sous pression réduite pour donner 3,3 g du composé de formule (I) pour lequel R₁-(R₂)ₘ-(R₃)ₙ-(R₄)ₚ représente Fmoc, R, R₅ et R₆ représentent des radicaux hydroxy et R₇ représente un atome d'hydrogène sous la forme d'une poudre blanche [Spectre de masse sur Sciex API III en ESMS (Electrospray Mass Spectrometry) : M/z = 861, (M+Na)⁺, M/z = 839 (M+H)⁺; Spectre infrarouge (FMIR dans le méthanol) : 3395, 3335, 2970, 2940, 2880, 3125 à 2125, 1700, 1665, 1590, 1500, 1450, 1225; Spectre de RMN (400MHz; DMSO; TEMP = 297K, δ en ppm) : R-Fmoc-1 : HA1,HA2 4,23; HB 4,48; HG1, HG2 7,70; HD1, HD2 7,37; HE1, HE2: 7,45; HZ1, HZ2 7,92; X-TYR1 : NH 5,73; HA 4,52; HB1, HB2 2,93, 3,03; HD1 6,70; HD2 6,72 HE1 6,70; X-ILE 2 : NH 8,36; HA 4,12; HB 1,62; MG1 0,83; HG21, HG22 1,18, 1,49; MD 0,83; X-TYR3 : NH 8,67; HA 3,58; HB1, HB2 2,45; 2,79; HD1 6,57; HD2 5,51; X-TYR4 : NH 4,17; HA 4,28; HB1, HB2 3,18, 3,43; HD1 7,19; HD2 7,31; HE1 7,52; HE2 6,80].

### EXEMPLE 14

On traite 100 mg de Fmoc-Arg(Pmc)-Arg(Pmc)-Pro-OH (85 µmol) par 17,4 mg de dicyclohexylcarbodiimide (85 µmol) et 12,2 mg de N-hydroxybenzotriazole (90 µmol) dans 1,5 ml de diméthylformamide pendant 4 heures à une température voisine de 20°C. La dicyclohexylurée formée est ensuite filtrée sur un filtre Millex HV 0,45 µm (Millipore) et le filtre est lavé par 0,5 ml de diméthylformamide. On ajoute ensuite 0,972 ml du filtrat obtenu (correspondant à 41 µmol de Fmoc-Arg(Pmc)-Arg(Pmc)-Pro-OH) à 0,5 ml d'une solution contenant 32 mg (48,6 µmol) du composé de formule (II) pour lequel R, R' et R" représentent des radicaux méthoxy et R"' représente un atome d'hydrogène et 7 µl (40 µmol) de diisopropyléthylamine. La réaction est conduite pendant 3 heures à une température voisine de 20°C. Le solvant est ensuite éliminé grâce à un évaporateur centrifuge Speed Vac (Savant) équipé d'une pompe à palettes pendant 18 heures à une température voisine de 20°C. Le résidu est ensuite repris par 10 ml d'un mélange acide trifluoroacétique-phénol-ethanedithiol-thioanisole-eau (40/3/1/2/2 en volumes) et laissé à réagir pendant 90 minutes à une température voisine de 20°C sous agitation. Le mélange est ensuite concentré sur un évaporateur centrifuge RC10-10 (Jouan) équipé d'une pompe à palettes pendant 45 minutes (température de la chambre d'évaporation 50°C, piégeage des vapeurs à -90°C). Le concentrat obtenu (environ 2 ml ) est additionné de 30 ml d'un mélange éther tert-butylméthylique-éther de pétrole (1/1 en volumes) pour précipiter le peptide. Ce peptide est collecté par centrifugation. Il est solubilisé par 2 ml d'acide trifluoroacétique et l'opération de précipitation est répétée 1 fois. Le peptide est séché sous pression réduite (3,5 kPa). Le produit est enfin purifié par chormatographie liquide à haute performance (CLHP) sur une colonne C18 100 Å (250 x 10 mm, Bio-Rad), élué par un gradient de 22 à 47% d'acétonitrile contenant 0,07% d'acide trifluoroacétique (en volumes) dans l'eau contenant 0,07% d'acide trifluoroacétique (en volumes), à un débit de 6 ml/minute, en 30 minutes, puis lyophilisé. On obtient ainsi 23,6 mg de ditrifluoroacétate du composé de formule (I) pour lequel R₁-(R₂)ₘ-(R₃)ₙ-(R₄)ₚ représente Fmoc-Arg-Arg-Pro-, R, R₅ et R₆ représentent des radicaux méthoxy et R₇ représente un atome d'hydrogène sous la forme d'une poudre blanche [Spectre de masse, sur Sciex API III en ESMS (Electrospray Mass Spectrometry) : M/z = 1290 (M+H)⁺, M/z = 646 (M+2H)²⁺].

Le composé de formule (II) pour lequel R, R' et R" représentent des radicaux méthoxy et R"' représente un atome d'hydrogène peut être obtenu de la manière suivante : sous atmosphère inerte, à une solution de 1,4 g du composé de formule (I) pour lequel R, R₅ et R₆ représentent des radicaux hydroxy, R₇ représente un atome d'hydrogène et R₁-(R₂)ₘ-(R₃)ₙ-(R₄)ₚ représente Fmoc- obtenu à l'exemple 13 dissous dans 30 ml de méthanol, on additionne en une heure 20 ml d'une solution 2M de triméthylsilyldiazométhane dans l'hexane et agite 18 heures à une température voisine de 20°C. On additionne à nouveau 10 ml d'une solution 2M de triméthylsilyldiazométhane dans l'hexane et agite 24 heures à une température voisine de 20°C. On additionne 0,5 ml d'acide acétique, et évapore les solvants sous pression réduite (2 kPa). Le résidu obtenu est purifié par chromatographie sur une colonne de 4 cm de diamètre contenant 250 g de gel de silice, en éluant successivement avec 750 ml de dichlorométhane, 3000 ml du mélange dichlorométhane-méthanol (98/2 en volumes), puis du mélange dichlorométhane-méthanol (95/5 en volumes), en recueillant des fractions de 100 ml. Les fractions comprises entre 2500 et 4400 ml sont jointes et évaporées pour donner 932 mg d'un solide blanc. On peut continuer à purifier le produit, en le dissolvant dans 10 ml de dichlorométhane, en diluant avec 50 ml d'éthanol; la solution est ensuite concentrée sous pression réduite à une température voisine de 20°C, jusqu'à début de précipitation, et laissée au repos 16 heures à une température voisine de +5°C. On obtient ainsi 441 mg du composé de formule (I) pour lequel R, R₅ et R₆ représentent des radicaux méthoxy et R₇ représente un atome d'hydrogène et R₁-(R₂)ₘ-(R₃)ₙ-(R₄)ₚ représente Fmoc, sous forme d'une poudre blanche fondant à 145°C, en se décomposant [Spectre de masse sur Autospec VG FISONS, en LIMS (Liquid Secondary Ion Mass Spectroscopy), matrice de glycérol+thioglycérol: M/z = 881 (MH)⁺]. A 187 mg du composé précédemment obtenu dissous dans 20 ml de dichlorométhane, on ajoute 0,1 ml de pipéridine et agite à une température voisine de 20°C pendant 42 heures. Les solvants sont évaporés sous pression réduite (2 kPa); le résidu est trituré dans 10 ml de diéthyléther, à une température voisine de 35°C, refroidi à une température voisine de 20°C, filtré et lavé trois fois avec 5 ml de diéthyléther pour donner 140 mg de poudre blanche qui est reprise dans 5 ml d'eau et extraite cinq fois avec 20 ml d'acétate d'éthyle. Les solvants sont évaporés sous pression réduite (2 kPa). La purification est poursuivie par reprise dans 1 ml de dichlorométhane dilué avec 10 ml de diéthyléther. On obtient ainsi 80 mg du composé de formule (II) pour lequel R, R₅ et R₆ représentent des radicaux méthoxy et R₇ représente un atome d'hydrogène sous la forme d'une poudre légèrement jaune, fondant à 240°C, en se décomposant [Spectre de masse, sur Autospec VG FISONS, en LIMS (Liquid Secondary Ion Mass Spectroscopy), matrice de glycérol+thioglycérol: M/z = 659 (MH)⁺].

Les médicaments selon l'invention sont constitués par un composé de formule (I) sous forme libre ou sous forme d'un sel pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles dans le traitement et la prévention des désordres associés à la neurotensine. C'est ainsi que ces composés sont utiles pour le traitement ou la prévention des psychoses, des troubles anxieux, de la dépression, des troubles cognitifs, de la neurodégénération, des attaques de panique, de la maladie de Parkinson, de la maladie d'Alzheimer, de la schizophrénie, de l'autisme, de la diskynésie tardive, du syndrôme du colon irritable, de la pancréatite aiguë, des ulcères, des désordres de la motilité intestinale, de certaines tumeurs sensibles à la neurotensine, dans le sevrage aux traitements chroniques et abus d'alcool ou de médicaments, des phénomènes allergiques et inflammatoires et des troubles cardiovasculaires et respiratoires.

Les doses dépendent de l'effet recherché, de la durée du traitement et de la voie d'administration utilisée; elles sont généralement comprises entre 50 et 300 mg par jour par voie orale pour un adulte avec des doses unitaires allant de 10 à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p. 4 cm3

## Revendications

1. Composés de formule : 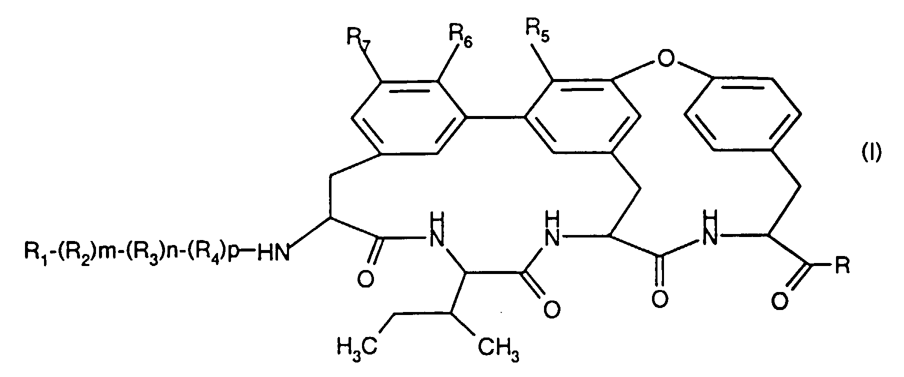 dans laquelle
R représente un radical hydroxy, alkyloxy, phénylalkyloxy ou -NH-CH₂-COOH,
R₁ représente un atome d'hydrogène, un radical adamantylacétyle, adamantylcarbo nyle, norbornylacétyle, norbornylphénoxycarbonyle, benzoyle, nicotinoyle, 4-phénylbenzoyle, 4-tert-butylbenzoyle, 2-pyrrolidinecarbonyle ou un groupe protecteur d'une fonction amine,
R₂ représente un résidu Arg ou Lys,
R₃ représente un résidu Arg ou Lys,
R₄ représente un résidu Pro,
m, n et p, identiques ou différents représentent un nombre égal à 0 ou 1, étant entendu que lorsque R₁ est un atome d'hydrogène, la somme m+n+p est au moins égale à 1
R₅ et R₆ sont identiques et représentent un radical hydroxy ou méthoxy et
R₇ représente un atome d'hydrogène, de chlore, de brome ou d'iode ou un radical nitro,
et leurs équivalents dans lesquels une ou plusieurs liaisons peptidiques (CO-NH) entre deux résidus d'acide aminé sont remplacées par des liaisons -CH₂-NH et/ou la liaison peptidique (CO-NH) entre les résidus d'acide aminé R₂ et R₃ est remplacée par une liaison CH=CH,
étant entendu que les radicaux et portions alkyle et alkyloxy contiennent 1 à 4 atomes de carbone en chaîne droite ou ramifiée, Arg signifie arginine, Lys signifie lysine, Pro signifie proline,
et leurs sels.

2. Composés de formule (I) selon la revendication 1 pour lesquels le groupe protecteur de la fonction amine représentant R₁ est un groupe 9-fluorénylméthoxycarbonyle, tertbutoxycarbonyle, acétyle, pivaloyle et benzyloxycarbonyle dont le phényle est éventuellement substitué par halogène, alkyle, alkyloxy ou nitro.

3. procédé de préparation des composés de formule (I) selon la revendication 1 pour lesquels la somme m+n+p est égale à au moins 1 caractérisé en ce que l'on fait réagir un dérivé de formule : 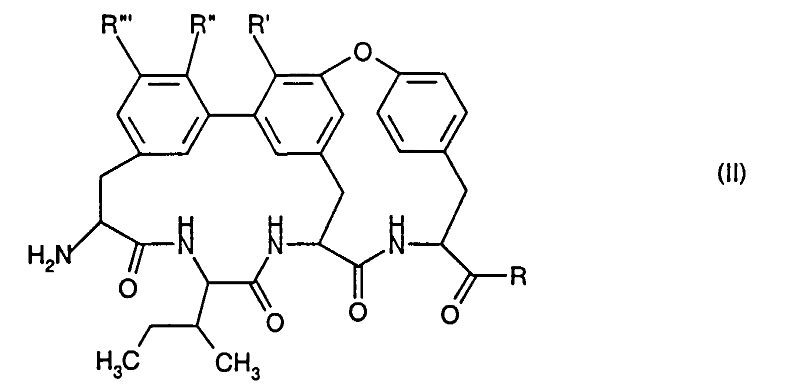 dans laquelle R a les mêmes significations que dans la revendication 1, R' et R" sont identiques et représentent chacun un radical hydroxy ou méthoxy et R"' représente un atome d'hydrogène, de chlore, de brome ou d'iode ou un radical nitro sur un dérivé de formule :
R₁-(R₂)m-(R₃)n-(R₄)p-OH (III)
dans laquelle R₁ représente un radical adamatylacétyle, adamantylcarbonyle, norbornylacétyle, norbornylphénoxycarbonyle, benzoyle, nicotinoyle, 4-phénylbenzoyle, 4-tert-butylbenzoyle, 2-pyrrolidinecarbonyle ou un groupe protecteur d'une fonction amine, R₂, R₃, R₄, n, m et p ont les mêmes significations que dans la revendication 1, la somme m+n+p étant égale à au moins 1, éventuellement suivie d'une déprotection de la fonction amine terminale pour obtenir les composés pour lesquels R₁ représente un atome d'hydrogène et/ou éventuellement lorsque R représente un radical alkyloxy ou phénylalkyloxy, d'une déprotection de la fonction carboxy pour obtenir les composés pour lesquels R représente un radical hydroxy, isole le produit et le transforme éventuellement en sel.

4. Procédé de préparation des composés de formule (I) selon la revendication 1 pour lesquels la somme m+n+p est au moins égale à 1, R₁ représente un atome d'hydrogène ou un groupe protecteur d'une fonction amine caractérisé en ce que l'on fait réagir un dérivé de formule (II) selon la revendication 3 sur successivement un dérivé (R₄)p-OH et/ou (R₃)n-OH et/ou (R₂)m-OH dans lesquels R4, R₃ et R₂ ont les mêmes significations que dans la formule (I) dont les restes amino sont de préférence protégés suivie éventuellement d'une déprotection, isole le produit et le transforme éventuellement en sel.

5. Procédé de préparation des composés de formule (I) selon la revendication 1 pour lesquels la somme m+n+p est égale à 0, R₁ représente un radical 9-fluorénylméthoxycarbonyle caractérisé en ce que l'on fait réagir un dérivé de formule (II) selon la revendication 3 sur le carbonate de 9-fluorénylméthyle et de N-succinyle, isole le produit et le transforme éventuellement en sel.

6. Procédé de préparation des composés de formule (I) selon la revendication 1 pour lesquels R₁ représente un radical tertbutoxycarbonyle, acétyle, pivaloyle et benzyloxycarbonyle dont le phényle est éventuellement substitué par halogène, alkyle, alkyloxy ou nitro caractérisé en ce que l'on fait réagir un dérivé de formule (II) selon la revendication 3 sur un dérivé R₁-Cl dans lequel R₁ représente un radical tertbutoxycarbonyle, acétyle, pivaloyle ou benzyloxycarbonyle dont le phényle est éventuellement substitué par halogène, alkyle, alkyloxy ou nitro, isole le produit et le transforme éventuellement en sel.

7. Procédé de préparation des composés de formule (I) selon la revendication 1 pour lesquels la somme m+n+p est égale à zéro et R₁ représente un radical adamantylacétyle, adamantylcarbonyle, norbornylacétyle, norbornylphénoxycarbonyle, benzoyle, nicotinoyle, 4-phénylbenzoyle, 4-tert-butylbenzoyle, 2-pyrrolidinecarbonyle caractérisé en ce que l'on fait réagir un dérivé de formule (II) selon la revendication 3 pour lequel R₁ représente un atome d'hydrogène sur un chlorure R₁-Cl dans lequel R₁ représente un radical adamantylacétyle, adamantylcarbonyle, norbornylacétyle, norbornylphénoxycarbonyle, benzoyle, nicotinoyle, 4-phénylbenzoyle, 4-tert-butylbenzoyle, 2-pyrrolidinecarbonyle, isole le produit et le transforme éventuellement en sel.

8. Compositions pharmaceutiques contenant en tant que principe actif au moins un composé de formule (I) selon la revendication 1 ou un sel d'un tel composé.

## Claims

1. Compounds of formula: 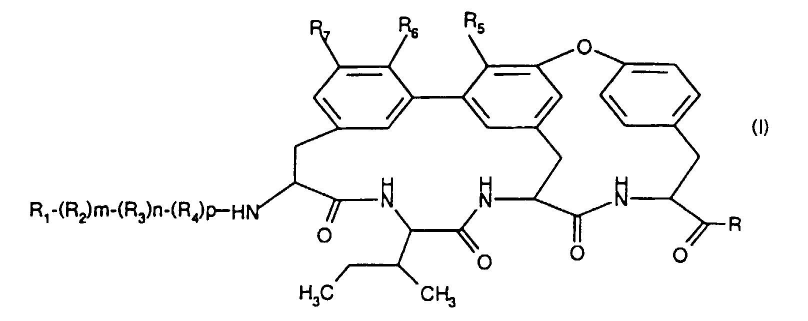 in which
R represents a hydroxyl, alkyloxy, phenylalkyloxy or -NH-CH₂-COOH radical,
R₁ represents a hydrogen atom, an adamantylacetyl, adamantylcarbonyl, norbornylacetyl, norbornylphenoxycarbonyl, benzoyl, nicotinoyl, 4-phenylbenzoyl, 4-tert-butylbenzoyl or 2-pyrrolidinylcarbonyl radical or a protective group for an amine functional group,
R₂ represents an Arg or Lys residue,
R₃ represents an Arg or Lys residue,
R₄ represents a Pro residue,
m, n and p, which are identical or different, represent a number equal to 0 or 1, it being understood that, when R₁ is a hydrogen atom, the sum m+n+p is at least equal to 1,
R₅ and R₆ are identical and represent a hydroxyl or methoxy radical and
R₇ represents a hydrogen, chlorine, bromine or iodine atom or a nitro radical,
and their equivalents in which one or a number of the peptide bonds (CO-NH) between two amino acid residues are replaced by -CH₂-NH- bonds and/or the peptide bond (CO-NH) between the R₂ and R₃ amino acid residues is replaced by a -CH=CH- bond,
it being understood that the alkyl and alkyloxy radicals and portions contain 1 to 4 carbon atoms in a straight or branched chain, Arg means arginine, Lys means lysine and Pro means proline,
and their salts.

2. Compounds of formula (I) according to claim 1 in which the protective group for the amine functional group representing R₁ is a 9-fluorenylmethoxycarbonyl, tert-butoxycarbonyl, acetyl, pivaloyl and benzyloxycarbonyl group, the phenyl in the benzyloxycarbonyl group being optionally substituted by halogen, alkyl, alkyloxy or nitro.

3. Process for the preparation of the compounds of formula (I) according to claim 1 in which the sum m+n+p is equal to at least 1, characterized in that a derivative of formula: 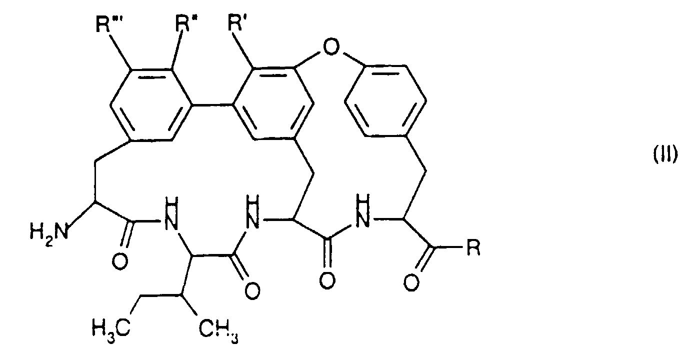 in which R has the same meanings as in claim 1, R' and R" are identical and each represent a hydroxyl or methoxy radical and R"' represents a hydrogen, chlorine, bromine or iodine atom or a nitro radical, is reacted with a derivative of formula:
R₁-(R₂)m-(R₃)n-(R₄)p-OH (III)
in which R₁ represents an adamantylacetyl, adamantylcarbonyl, norbornylacetyl, norbornylphenoxycarbonyl, benzoyl, nicotinoyl, 4-phenylbenzoyl, 4-tert-butylbenzoyl or 2-pyrrolidinylcarbonyl radical or a protective group for an amine functional group and R₂, R₃, R₄, n, m and p have the same meanings as in claim 1, the sum m+n+p being equal to at least 1, optionally followed by deprotection of the end amine functional group in order to obtain the compounds in which R₁ represents a hydrogen atom and/or optionally, when R represents an alkyloxy or phenylalkyloxy radical, by deprotection of the carboxyl functional group in order to obtain the compounds in which R represents a hydroxyl radical, the product is isolated and is optionally converted to a salt.

4. Process for the preparation of the compounds of formula (I) according to claim 1 in which the sum m+n+p is at least equal to 1 and R₁ represents a hydrogen atom or a protective group for an amine functional group, characterized in that a derivative of formula (II) according to claim 3 is reacted successively with a derivative (R₄)ₚ-OH and/or (R₃)ₙ-OH and/or (R₂)ₘ-OH in which R₄, R₃ and R₂ have the same meanings as in the formula (I), the amino residues of which are preferably protected, optionally followed by a deprotection, the product is isolated and is optionally converted to a salt.

5. Process for the preparation of the compounds of formula (I) according to claim 1 in which the sum m+n+p is equal to 0 and R₁ represents a 9-fluorenylmethoxycarbonyl radical, characterized in that a derivative of formula (II) according to claim 3 is reacted with 9-fluorenylmethyl N-succinyl carbonate, the product is isolated and is optionally converted to a salt.

6. Process for the preparation of the compounds of formula (I) according to claim 1 in which R₁ represents a tert-butoxycarbonyl, acetyl, pivaloyl or benzyloxycarbonyl radical in which the phenyl is optionally substituted by halogen, alkyl, alkyloxy or nitro, characterized in that a derivative of formula (II) according to claim 3 is reacted with a derivative R₁-Cl in which R₁ represents a tert-butoxycarbonyl, acetyl, pivaloyl or benzyloxycarbonyl radical in which the phenyl is optionally substituted by halogen, alkyl, alkyloxy or nitro, the product is isolated and is optionally converted to a salt.

7. Process for the preparation of the compounds of formula (I) according to claim 1 in which the sum m+n+p is equal to zero and R₁ represents an adamantylacetyl, adamantylcarbonyl, norbornylacetyl, norbornylphenoxycarbonyl, benzoyl, nicotinoyl, 4-phenylbenzoyl, 4-tert-butylbenzoyl or 2-pyrrolidinylcarbonyl radical, characterized in that a derivative of formula (II) according to claim 3 in which R₁ represents a hydrogen atom is reacted with a chloride R₁-Cl in which R₁ represents an adamantylacetyl, adamantylcarbonyl, norbornylacetyl, norbornylphenoxycarbonyl, benzoyl, nicotinoyl, 4-phenylbenzoyl, 4-tert-butylbenzoyl or 2-pyrrolidinylcarbonyl radical, the product is isolated and is optionally converted to a salt.

8. Pharmaceutical compositions containing, as active principle, at least one compound of formula (I) according to claim 1 or a salt of such a compound.

## Patentansprüche

1. Verbindungen der Formel: 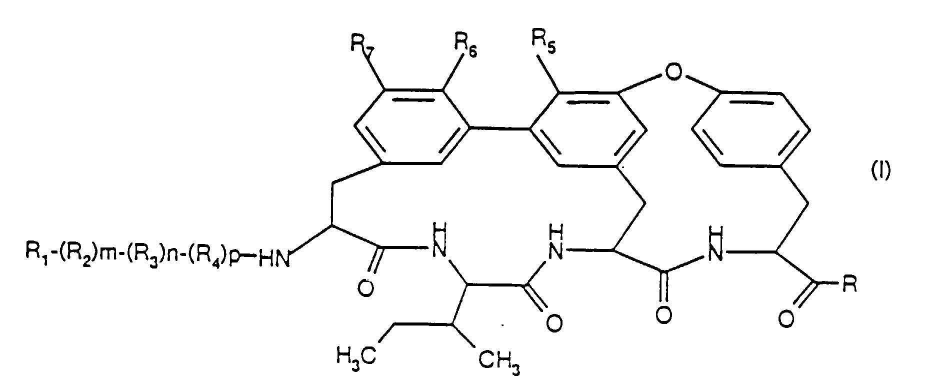 worin
R für einen Hydroxy-, Alkyloxy-, Phenylalkyloxyrest oder -NH-CH₂-COOH steht,
R₁ für ein Wasserstoffatom, eine Adamantylacetyl-, Adamantylcarbonyl-, Norbornylacetyl-, Norbornylphenoxycarbonyl-, Benzoyl-, Nicotinoyl-, 4-Phenylbenzoyl-, 4-tert.-Butylbenzoyl-, 2-Pyrrolidincarbonylgruppe oder eine Schutzgruppe einer Aminfunktion steht,
R₂ einen Arg- oder Lys-Rest bedeutet,
R₃ einen Arg- oder Lys-Rest bedeutet,
R₄ einen Pro-Rest bedeutet,
m, n und p, die gleich oder verschieden sind, eine ganze Zahl von 0 oder 1 bedeuten, mit der Maßgabe, daß, wenn R₁ ein Wasserstoffatom ist, die Summe m+n+p mindestens gleich 1 ist,
R₅ und R₆ gleich sind und für eine Hydroxy- oder Methoxygruppe stehen, und
R₇ ein Wasserstoff-, Chlor-, Brom- oder Iodatom oder eine Nitrogruppe bedeutet,
und ihre Äquivalente, in denen eine oder mehrere Peptidbindungen (CO-NH) zwischen zwei Aminosäureresten durch Bindungen -CH₂-NH ersetzt sind und/oder die Peptidbindung (CO-NH) zwischen den Aminosäureresten R₂ und R₃ durch eine Bindung CH=CH ersetzt ist,
mit der Maßgabe, daß die Alkyl- und Alkyloxyreste und -anteile 1 bis 4 Kohlenstoffatome in gerader oder verzweigter Kette enthalten, Arg Arginin bedeutet, Lys Lysin bedeutet, Pro Prolin bedeutet,
und ihre Salze.

2. Verbindungen der Formel (I) nach Anspruch 1, in denen die Schutzgruppe der Aminfunktion, die durch R₁ wiedergegeben wird, eine 9-Fluorenylmethoxycarbonyl-, tert.-Butoxycarbonyl-, Acetyl-, Pivaloyl- und Benzyloxycarbonylgruppe ist, wobei der Phenylrest gegebenenfalls durch Halogen, Alkyl, Alkyloxy oder Nitro substituiert ist.

3. Verfahren zur Herstellung von Verbindungen der Formel (I) nach Anspruch 1, worin die Summe m+n+p mindestens gleich 1 ist, dadurch **gekennzeichnet**, daß man ein Derivat der Formel: 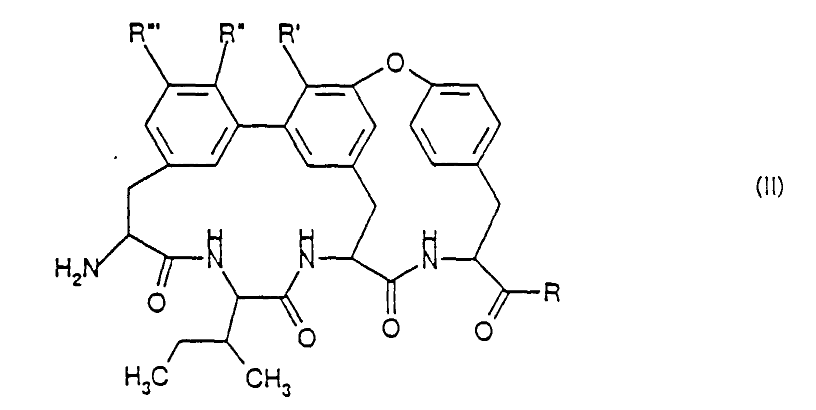 worin R wie in Anspruch 1 definiert ist, R' und R" gleich sind und jeweils eine Hydroxy- oder Methoxygruppe bedeuten und R"' ein Wasserstoff-, Chlor-, Brom- oder Iodatom oder eine Nitrogruppe bedeutet, mit einem Derivat der Formel:
R₁-(R₂)m-(R₃)n-(R₄)p-OH (III)
worin R₁ eine Adamantylacetyl-, Adamantylcarbonyl-, Norbornylacetyl-, Norbornylphenoxycarbonyl-, Benzoyl-, Nicotinoyl-, 4-Phenylbenzoyl-, 4-tert.-Butylbenzoyl-, 2-Pyrrolidincarbonylgruppe oder eine Schutzgruppe einer Aminfunktion bedeutet, R₂, R₃, R₄, n, m und p wie in Anspruch 1 definiert sind, die Summe m+n+p mindestens gleich 1 ist, umsetzt, gegebenenfalls gefolgt von einer Abspaltung der Schutzgruppe der terminalen Aminfunktion, um Verbindungen zu erhalten, in denen R₁ ein Wasserstoffatom bedeutet, und/oder gegebenenfalls, wenn R einen Alkyloxy- oder Phenylalkyloxyrest bedeutet, von einer Abspaltung der Schutzgruppe der Carboxyfunktion, um Verbindungen zu erhalten, in denen R eine Hydroxygruppe bedeutet, die Verbindung isoliert und gegebenenfalls in ein Salz überführt.

4. Verfahren zur Herstellung von Verbindungen der Formel (I) nach Anspruch 1, in denen die Summe m+n+p mindestens gleich 1 ist, R₁ ein Wasserstoffatom oder eine Schutzgruppe einer Aminfunktion bedeutet, dadurch **gekennzeichnet**, daß man ein Derivat der Formel (II) nach Anspruch 3 sukkzessive mit einem Derivat (R₄)p-OH und/oder (R₃)n-OH und/oder (R₂)m-OH umsetzt, worin R₄, R₃ und R₂ wie für Formel (I) definiert sind, deren Aminogruppen bevorzugt geschützt sind, gegebenenfalls gefolgt von einer Abspaltung der Schutzgruppe, die Verbindung isoliert und gegebenenfalls in ein Salz überführt.

5. Verfahren zur Herstellung von Verbindungen der Formel (I) nach Anspruch 1, in denen die Summe m+n+p gleich 0 ist, R₁ eine 9-Fluorenylmethoxycarbonylgruppe bedeutet, dadurch **gekennzeichnet**, daß man ein Derivat der Formel (II) nach Anspruch 3 mit 9-Fluorenylmethyl-N-succinylcarbonat umsetzt, die Verbindung isoliert und gegebenenfalls in ein Salz überführt.

6. Verfahren zur Herstellung von Verbindungen der Formel (I) nach Anspruch 1, in denen R₁ eine tert.-Butoxycarbonyl-, Acetyl-, Pivaloyl- und Benzyloxycarbonylgruppe bedeutet, worin der Phenylrest gegebenenfalls durch Halogen, Alkyl, Alkyloxy oder Nitro substituiert ist, dadurch **gekennzeichnet**, daß man ein Derivat der Formel (II) nach Anspruch 3 mit einem Derivat R₁-Cl, worin R₁ eine tert.-Butoxycarbonyl-, Acetyl-, Pivaloyl- oder Benzyloxycarbonylgruppe bedeutet, wobei die Phenylgruppe gegebenenfalls durch Halogen, Alkyl, Alkyloxy oder Nitro substituiert ist, umsetzt, die Verbindung isoliert und gegebenenfalls in ein Salz überführt.

7. Verfahren zur Herstellung von Verbindungen der Formel (I) nach Anspruch 1, in denen die Summe m+n+p gleich null ist und R₁ eine Adamantylacetyl-, Adamantylcarbonyl-, Norbornylacetyl-, Norbornylphenoxycarbonyl-, Benzoyl-, Nicotinoyl-, 4-Phenylbenzoyl-, 4-tert.-Butylbenzoyl-, 2-Pyrrolidincarbonylgruppe bedeutet, dadurch **gekennzeichnet**, daß man ein Derivat der Formel (II) nach Anspruch 3, in dem R₁ ein Wasserstoffatom bedeutet, mit dem Chlorid R₁-Cl, worin R₁ eine Adamantylacetyl-, Adamantylcarbonyl-, Norbornylacetyl-, Norbornylphenoxycarbonyl-, Benzoyl-, Nicotinoyl-, 4-Phenylbenzoyl-, 4-tert.-Butylbenzoyl-, 2-Pyrrolidincarbonylgruppe bedeutet, umsetzt, die Verbindung isoliert und gegebenenfalls in ein Salz überführt.

8. Pharmazeutische Präparate, enthaltend als Wirkstoff mindestens eine Verbindung der Formel (I) nach Anspruch 1 oder ein Salz einer derartigen Verbindung.
